# EUROPÄISCHE PATENTSCHRIFT

(11) **EP 0 073 061 B1**
(45) Veröffentlichungstag und Bekanntmachung des Hinweises auf die Patenterteilung: **04.03.1987**
(21) Anmeldenummer: 82107790.6
(22) Anmeldetag: 25.08.1982
(51) Int. Cl.: C07D 205/08, C07D 405/14, C07D 405/04, C07D 403/04, C07D 493/14, C07D 317/28, C07D 417/12, C07D 277/40

(54) **Optisch einheitliche Beta-Lactame, deren Herstellung und Verwendung bei der Herstellung von antimikrobiell wirksamen Beta-Lactamen sowie Vorprodukte verwendbar zu deren Herstellung**
Optically uniform beta-lactams, their preparation and use in the preparation of antimicrobial beta-lactams, and starting compounds for use in their preparation
Bêta-lactames optiquement uniformes, leur préparation et application à la préparation de bêta-lactames antimicrobiennes ainsi que les produits de départ pour leur préparation

(30) Priorität: 27.08.1981 CH 5524/81; 25.06.1982 CH 3925/82
(43) Veröffentlichungstag der Anmeldung: 02.03.1983
(60) Teilanmeldung: 84115022.0
(73) Patentinhaber: F. HOFFMANN-LA ROCHE & CO. Aktiengesellschaft, 4002 Basel (CH)
(72) Erfinder: Hubschwerlen, Christian Nicolas, Dr., F-68200 Durmenach (FR); Schmid, Gérard, Dr., CH-4468 Kienberg (CH)
(74) Vertreter: Lederer, Franz, Dr.

## Beschreibung

Die vorliegende Erfindung betrifft β-Lactame und zwar optische einheitliche ß-Lactame der allgemeinen Formel 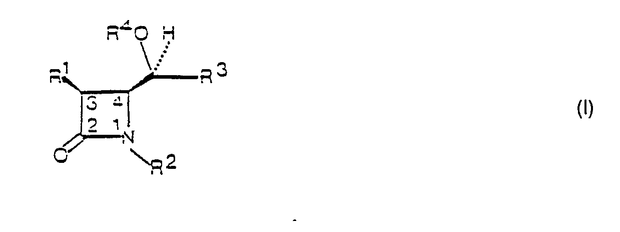 worin R¹ Amino, Azido, Phthalimido oder die Gruppe ROCO-CH=C(CH₃)-NH- oder Z-NH-, R niederes Alkyl, Z gegebenenfalls durch Chlor substituiertes niederes Alkoxycarbonyl, Benzyloxycarbonyl oder Benzhydryl, R² Wasserstoff oder die Gruppe Z'-, Z'-2,4- oder 3,4-Di(niederes Alkoxy)benzyl, 2,4-oder 3,4-Di(niederes Alkoxy)phenyl, Di[4-(niederes Alkoxy)-phenyl]methyl oder 4-(niederes Alkoxy)phenyl, R³ niederes Alkyl, Phenyl-niederes Alkyl, niederes Alkoxy-niederes Alkyl, insbesondere niederes Alkoxymethyl, und R⁴ niederes Alkyl oder Phenyl-niederes Alkyl oder R³ und R⁴ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden 0-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, bedeuten, wobei R¹ eine Gruppe der Formel Z-NH- bedeutet, wenn R² Wasserstoff bedeutet, die entsprechenden optischen Antipoden davon und die Herstellung dieser Verbindungen.

Gegenstand der vorliegenden Erfindung sind ferner optisch einheitliche β-Lactame der allgemeinen Formeln 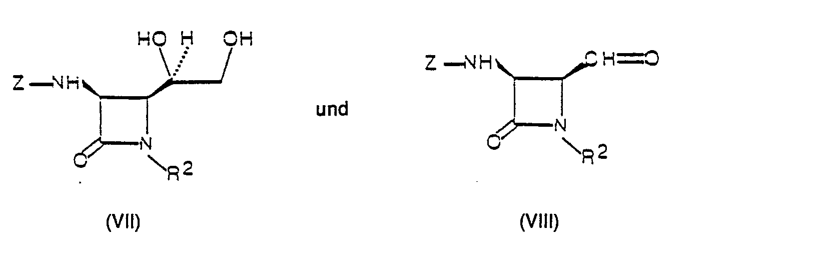 worin Z und R² obige Bedeutung besitzen, die entsprechenden optischen Antipoden davon und die Herstellung dieser Verbindungen, sowie die Herstellung von optisch einheitlichen β-Lactamen der allgemeinen Formel 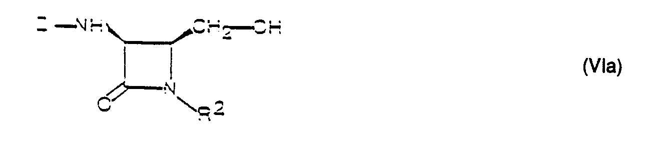 worin Z und R² obige Bedeutung besitzen, und der entsprechenden optischen Antipoden davon.

Der Ausdruck « niederes Alkyl für sich allein genommen, oder in Kombinationen wie « niederes Alkoxycarbonyl •, « niederes Alkoxy und dergleichen, bezeichnet geradkettige oder verzweigte gesättigte Kohlenwasserstoffreste mit höchstens 7, vorzugsweise höchstens 4 Kohlenstoffatomen, wie Methyl, Aethyl, Isopropyl und dergleichen. t-Butoxycarbonyl und Trichloräthoxycarbonyl sind Beispiele für gegebenenfalls durch Chlor substituiertes niederes Alkoxycarbonyl. Z' bedeutet beispielsweise 2,4-oder 3,4-Dimethoxybenzyl, 2,4- oder 3,4-Dimethoxyphenyl, Di(4-methoxyphenyl)methyl oder 4-Methoxyphenyl. Vorzugsweise bedeuten R³ und R⁴ zusammen mit dem Chiralitätszentrum eine Gruppe der Formel

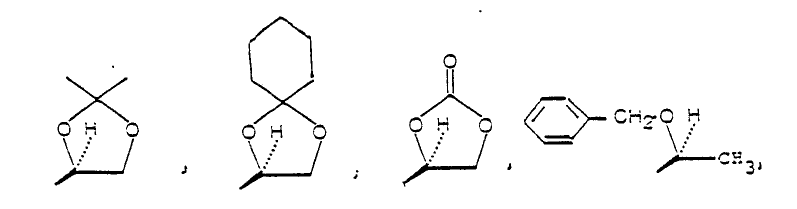

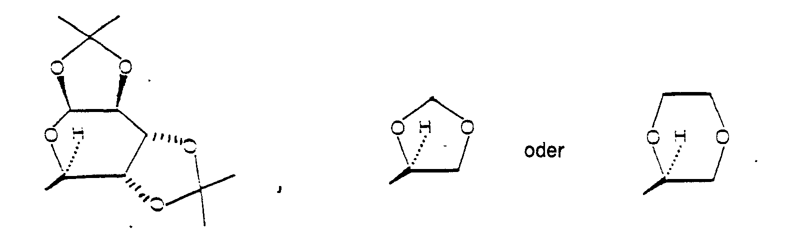

In einer speziellen Ausführungsform bedeuten R³ und R⁴ zusammen mit dem Chiralitätszentrum die Gruppe

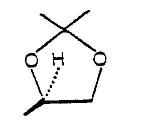

In einer weiteren speziellen Ausführungsform umfasst die vorliegende Erfindung Verbindungen der obigen Formel I, worin R¹ Phthalimido, Amino oder enzyloxycarbonylamino, R² Wasserstoff oder 2,4-Dimethoxybenzyl und R³ und R⁴ zusammen mit dem Chiralitätszentrum die Gruppe 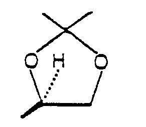 bedeuten, sowie die optischen Antipoden davon.

Die nachfolgend aufgeführten Verbindungen sind Vertreter der von der obigen Formel I umfassten Verbindungsklasse :
N-[(2R,3R)-1-(2,4-dimethoxybenzyl)-4-[(S)-1,4-dioxaspiro-[4.5]dec-2-yl]-2-oxo-3-azetidinyl]phthali- mid,
N-[(3S,4S)-cis-1-(3,4-Dimethoxybenzyl)-4-[(R)-2,2-dimethyl-1,3-dioxolan-4-yl]-2-oxo-3-azetidi- nyl]phthalimid und
N-[(3S,4S)-4-[(S)-1-(Benzyloxy)äthyl]-1-(2,4-dimethoxybenzyl)-2-oxo-3-azetidinyl]phthalimid.

Die nachfolgend aufgeführten Verbindungen sind bevorzugte Vertreter der von der obigen Formel I umfassten Verbindungsklasse :
N-[(3S,4S)-cis-1-(2,4-Dimethoxybenzyl)-4-[(R)-2,2-dimethyl-1,3-dioxolan-4-yl]-2-oxo-3-azetidi- nyl]phthalimid,
(3S,4S)-cis-3-Amino-1-(2,4-dimethoxybenzyl)-4-[(R)-2,2-dimethyl-1,3-dioxolan-4-yl]-2-azetidinon,
Benzyl-(3S,4S)-cis-1-(2,4-dimethoxybenzyl)-4-[(R)-2,2-dimethyl-1,3-dioxolan-4-yl]-2-oxo-3-azeti- dincarbamat und
Benzyl-(3S,4S)-cis-4-[(R)-2,2-dimethyl-1,3-dioxolan-4-yl]-2-oxo-3-azetidincarbamat.

Die ß-Lactame der Formel und die entsprechenden optischen Antipoden davon können erfindungsgemäss hergestellt werden, indem man ein reaktives Derivat einer Carbonsäure der allgemeinen Formel 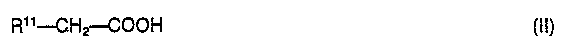 worin R" Azido, Phthalimido oder die Gruppe ROCO―CH=C(CH₃)―NH― und R niederes Alkyl bedeuten, in Gegenwart einer Base mit einer optisch einheitlichen Verbindung der allgemeinen Formel 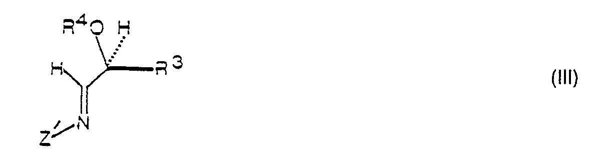 worin R³, R⁴ und Z' die obige Bedeutung besitzen oder dem optischen Antipoden davon umsetzt, erwünschtenfalls in einer so erhaltenen Verbindung der allgemeinen Formel 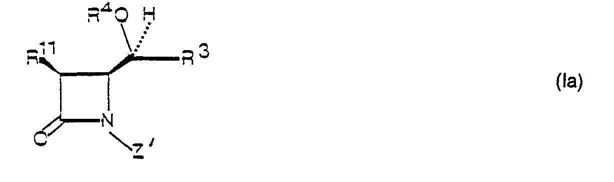 worin R", R³, R⁴ und Z' obige Bedeutung besitzen, oder dem optischen Antipoden davon die Gruppe R" in die Aminogruppe umwandelt, erwünschtenfalls eine so erhaltene Verbindung der allgemeinen Formel 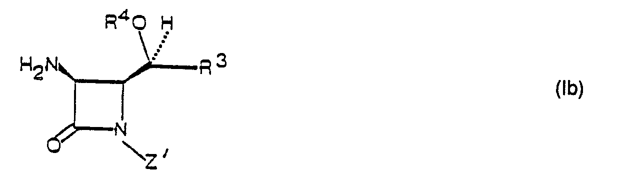 worin R³, R⁴ und Z' obige Bedeutung besitzen, oder den optischen Antipoden davon mit einem die Gruppe Z liefernden Mittel umsetzt und erwünschtenfalls in einer so erhaltenen Verfindung der allgemeinen Formel 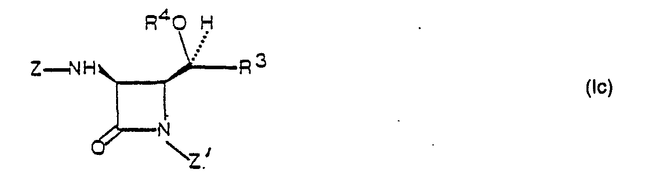 worin R³, R⁴, Z und Z' obige Bedeutung besitzen, oder dem optischen Antipoden davon die mit Z bezeichnete Gruppe abspaltet.

Bei der Reaktion eines reaktiven Derivates einer Carbonsäure der Formel II mit einer Verbindung der Formel III bzw. dem optischen Antipoden davon handelt es sich um eine dem Fachmann geläufige Cycloaddition. Geeignete reaktive Carbonsäurederivate sind beispielsweise die entsprechenden Carbonsäurehalogenide, insbesondere die Carbonsäurechloride, entsprechende Carbonsäureanhydride und gemischte Anhydride (z. B. Trifluoressigsäure, Mesitylensulfonsäure und dergleichen), entsprechende Carbonsäureimidazolide und dergleichen. Zweckmässigerweise arbeitet man dabei in Gegenwart einer Base, beispielsweise eines tertiären Amins, wie Triäthylamin, und in einem inerten organischen Lösungsmittel, wobei insbesondere Aether, wie Tetrahydrofuran, Diäthyläther, t-Butylmethyläther, Dioxan, Aethylenglykol, Dimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen in Frage kommen. Die obige Cycloaddition wird dabei in einem Temperaturbereich von etwa -30 °C bis etwa 50 °C durchgeführt.

Die obige Reaktion einer Verbindung der Formel II mit einer optisch einheitlichen Verbindung der Formel III oder dem optischen Antipoden davon liefert eine Verbindung der obigen Formel la bzw. den entsprechenden optischen Antipoden davon, wobei die Substituenten in 3- und 4-Stellung des Azetidinringes zueinander wie erwartet cis-ständig sind. Ueberraschenderweise hat es sich jedoch gezeigt, dass die Verwendung einer optisch aktiven Verbindung der Formel III bzw. des optischen Antipoden davon in der obigen Cycloaddition in hoher optischer Ausbeute zwei neue optische Zentren induziert, d. h. mit hoher Diastereoselektivität zur Bildung von nur einem von zwei möglichen diastereomeren Produkten führt. Im erhaltenen Produkt konnte das zweite mögliche cis-Cycloadditionsprodukt, das zum tatsächlich erhaltenen Produkt diastereoisomer wäre, nicht nachgewiesen werden.

Die als Ausgangsstoffe verwendeten Verbindungen der Formel 111 bzw. deren optische Antipoden können hergestellt werden, indem man den Aldehyd der Formel 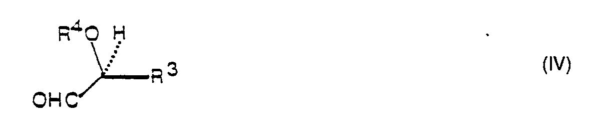 worin R³ und R⁴ obige Bedeutung besitzen, bzw. dessen optischen Antipoden mit einem Amin der allgemeinen Formel 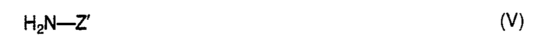 worin Z' obige Bedeutung besitzt, umsetzt. Man arbeitet dabei vorzugsweise in einem inerten organischen Lösungsmittel, beispielsweise in einem halogenierten Kohlenwasserstoff, wie Methylenchlorid, Chloroform, 1,2-Dichloräthan und dergleichen, oder in einem Kohlenwasserstoff, wie Benzol, Toluol und dergleichen. Vorzugsweise entfernt man dabei laufend das während der Reaktion entstehende Reaktionswasser, beispielsweise durch azeotrope Destillation oder durch Arbeiten in Gegenwart eines wasserentziehenden Mittels, beispielsweise in Gegenwart eines geeigneten Molekularsiebes, oder von anderen herkömmlichen Trocknungsmittels, wie Kaliumcarbonat, Magnesiumsulfat und dergleichen. Bei azeotroper Entfernung des gebildeten Reaktionswassers arbeitet man bei der Siedetemperatur des gewählten Lösungsmittels ; bei Verwendung eines wasserentziehenden Mittels arbeitet man vorzugsweise bei Raumtemperatur.

Die Verbindungen der Formel III und ihre entsprechenden optischen Antipoden, welche in der weiter oben beschriebenen Cycloaddition überraschenderweise nur das eine von zwei diastereomeren Produkten liefern, sind neu. Sie sind Gegenstand der Europäischen Patentanmeldung Nr. 841150220, einer Teilanmeldung aus vorliegender Anmeldung. Sie müssen nicht isoliert werden, sondern können direkt der erfindungsgemässen Cycloaddition unterworfen werden.

Die Verbindungen der Formel Ib und die optischen Antipoden davon können erfindungsgemäss dadurch erhalten werden, dass man in einer Verbindung der Formel la oder dem optischen Antipoden davon die Gruppe R¹¹ in die Aminogruppe umwandelt. Diese Reaktion erfolgt nach an sich bekannten und jedem Fachmann geläufigen Methoden, wobei die anzuwendende Methode von der Natur der Gruppe R" abhängt. Die Phthalimidogruppe kann beispielsweise durch Umsetzen mit Hydrazin, Methylhydrazin oder dergleichen entfernt werden, wobei man zweckmässigerweise in einem inerten organischen Lösungsmittel arbeitet. Geeignete Lösungsmittel sind z. B. halogenierte Kohlenwasserstoffe, wie Methylenchlorid, Chloroform und dergleichen, Aether, wie Tetrahydrofuran, Dioxan, t-Butylmethyläther und dergleichen, usw. Die Azidogruppe kann beispielsweise mit elementarem Wasserstoff in Gegenwart eines Katalysators, wie Palladium/Kohle, Raney-Nickel, Platinoxid und dergleichen, zur Aminogruppe reduziert werden. Die Gruppe ROCO―CH=C(CH₃)―NH― kann beispielsweise durch milde saure Hydrolyse in die Aminogruppe übergeführt werden.

Durch Behandeln einer Verbindung der Formel lb oder des optischen Antipoden davon mit einem die Gruppe Z liefernden Mittel erhält man erfindungsgemäss eine Verbindung der Formel Ic oder den optischen Antipoden davon. Geeignete, die Gruppe Z liefernde Mittel sind beispielsweise Di-t-butyldicarbonat oder Chlorameisensäureester, wie Chlorameisensäurebenzylester, Chlorameisensäure-t-butylester, Chlorameisensäure-2,2,2-trichloräthylester und dergleichen. Die Reaktion erfolgt zweckmässigerweise in einem inerten organischen Lösungsmittel, beispielsweise in einem halogenierten Kohlenwasserstoff, wie Methylenchlorid, Chloroform und dergleichen, und zweckmässigerweise in Gegenwart eines säurebindenden Mittels, wie Butylenoxid, Triäthylamin, Chinuclidin, usw. Zweckmässigerweise arbeitet man bei Raumtemperatur.

Durch Abspalten der mit Z bezeichneten Gruppe aus einer Verbindung der Formel lc bzw. aus dem optischen Antipoden davon erhält man eine Verbindung der allgemeinen Formel 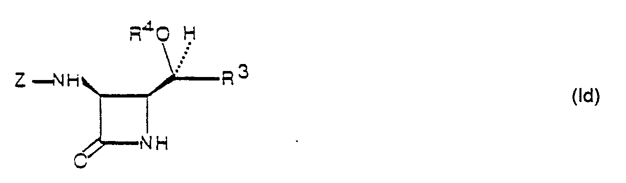 worin R³, R⁴ und Z obige Bedeutung besitzen, bzw. den entsprechenden optischen Antipoden davon.

Die Abspaltung der mit Z' bezeichneten Gruppe aus einer Verbindung der Formel Ic bzw. aus dem optischen Antipoden davon erfolgt zweckmässigerweise durch milde Oxidation. Ein geeignetes Oxidationsmittel ist Ceriumammoniumnitrat z. B. in wässrigem Acetonitril oder in wässrigem Aceton. 2,4- und 3,4-Di-(nieder-Alkoxy)-benzyl- sowie Di-[4-(nieder-Aikoxy)-phenyi]-methyt-gruppen können ebenfalls mit einem gepufferten Peroxodisulfat, z. B. Ammoniumperoxodisulfat/Ammoniak oder Kaliumperoxodisulfat/Dikaliumhydrogenphosphat, abgespalten werden, wobei man in Wasser und unter annähernd neutralen Bedingungen arbeitet. Di-[4-(nieder-Alkoxy)-phenyl]-methyl-gruppen können auch acidolytisch abgespalten werden, z. B. durch Einwirken von Trifluoressigsäure, Ameisensäure oder Aluminiumchlorid in einem inerten organischen Lösungsmittel, wie Methylenchlorid oder Anisol.

Die Verbindungen der Formel I und die entsprechenden optischen Antipoden davon sind wertvolle Zwischenprodukte für die Herstellung von pharmazeutischen Wirkstoffen. Sie können insbesondere für die Herstellung von antibiotisch wirksamen Isocephalosporinen verwendet werden. Solche Isocephalosporine und ihre Herstellung aus Verbindungen der allgemeinen Formel 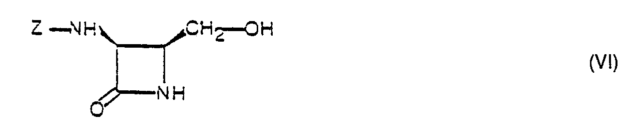 worin Z obige Bedeutung besitzt, werden in der deutschen Offenlegungsschrift Nr. 2 619 458 eingehend beschrieben.

Die Verbindungen der Formel VI und die entsprechenden optischen Antipoden davon können beispielsweise hergestellt werden, indem man in einer erhaltenen Verbindung der allgemeinen Formel 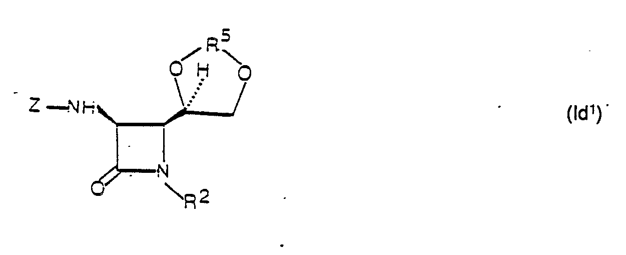 worin R⁵ Methylen, Aethylen, Oxomethylen, Cyclohexyliden oder Isopropyliden bedeutet und Z und R² die obige Bedeutung haben, bzw. dem optischen Antipoden davon den Rest R⁵ abspaltet, z. B. durch Behandeln mit einem mild sauren Agens, wie z. B. mit einem sulfonierten lonenaustauscher, Pyridinium-p-toluolsulfonat oder p-Toluolsulfonsäure, in einem niederen Alkanol, wie Methanol oder Aethanol, oder in wässrigem Tetrahydrofuran, vorzugsweise bei Ramtemperatur bis etwa 80 °C. Man erhält ein optisch aktives Diol der allgemeinen Formel 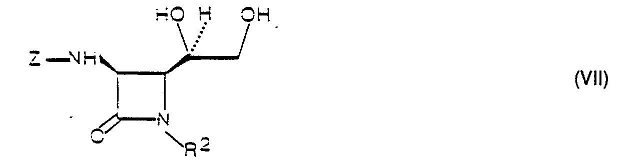 worin Z und R² die obige Bedeutung haben, bzw. den optischen Antipoden davon.

In der so erhaltenen Verbindung der Formel VII bzw. den optischen Antipoden davon wird die Diolgruppierung gespalten, wobei ein Aldehyd der allgemeinen Formel 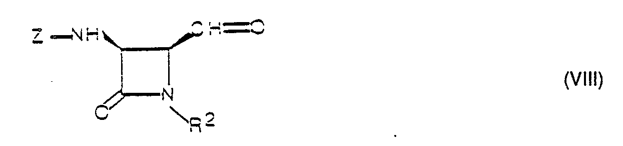 worin Z und R² die obige Bedeutung haben, bzw. den optischen Antipoden davon erhalten wird. Diese Spaltung erfolgt nach an sich bekannten Methoden und kann z. B. mit Alkalimetallperjodat, wie Natriummetaperjodat, in Wasser, ggfs. im Gemisch mit z. B. Tetrahydrofuran oder einem niederen Alkanol, wie Methanol, bewerkstelligt werden. Zur Ueberführung in den Alkohol der Formel Via bzw. den optischen Antipoden davon wird die Aldehydgruppe des Aldehyds VIII bzw. des optischen Antipoden davon nach bekannten Methoden reduziert, z. B. durch Behandeln mit einem komplexen Metallhydrid, wie Natriumborhydrid in einem niederen Alkanol, wie Aethanol, Isopropanol oder dgl. Ein erhaltener Alkohol der allgemeinen Formel 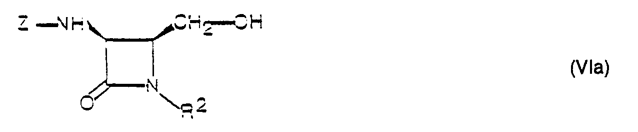 worin Z und R² die obigen Bedeutungen besitzen, bzw. der optische Antipode davon kann, sofern R² die Gruppe Z' darstellt, in obiger Weise durch milde Oxidation in den gewünschten, am Stickstoff nicht geschützten Alkohol der Formel VI bzw. den optischen Antipoden davon übergeführt werden.

Verbindungen der allgemeinen Formel 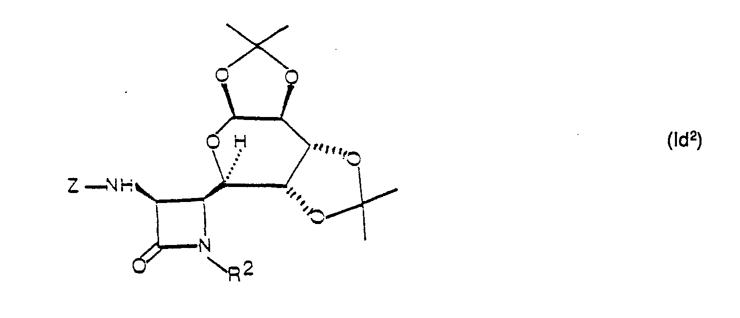 worin R² und Z die obige Bedeutung haben, bzw. die optischen Antipoden davon können durch saure Hydrolyse, z. B. mit Mineralsäure in einem niederen Alkanol (Wie Salzsäure in Methanol) und anschliessende Umsetzung mit wässrigem Natriummetaperjodat in die Aldehyde der Formel VIII bzw. die optischen Antipoden davon übergeführt werden.

Vorhandene Benzyloxygruppen, wie z. B. in Verbindungen der allgemeinen Formel 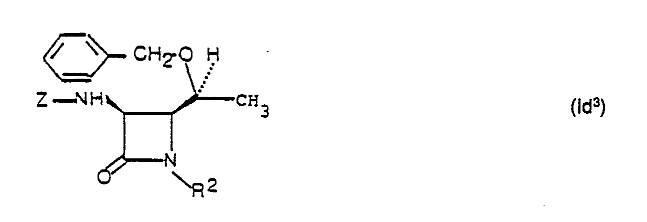 worin Z und R² die obige Bedeutung haben, bzw. in den optischen Antipoden davon können hydrogenolytisch gespalten und das erhaltene, entsprechende Carbinol der allgemeinen Formel 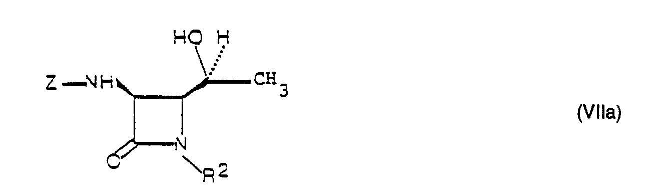 worin Z und R² die obige gegebene Bedeutung haben, bzw. der optische Antipode davon weiter umgesetzt werden, z. B. in Analogie zu Verbindung 9 im nachstehenden Schema II.

Die Verbindungen der Formel I bzw. die optischen Antipoden davon können durch Einführung geeigneter Reste in den Stellungen 1, 3 und 4 in optisch einheitliche β-Lactame mit antimikrobiellen Eigenschaften übergeführt werden. Der Substituent in Stellung 4 kann funktionell abgewandelt werden, z. B. durch Umwandlung der oben beschriebenen 4-Formyl- bzw. 4-Hydroxymethylgruppen in dem Fachmann bekannter Weise. Nach Entfernung einer allfälligen Schutzgruppe R² kann durch Umsetzung mit einem reaktionsfähigen Derivat von Schwefeltrioxid die Gruppe -S0₃H in Stellung 1 eingeführt werden, z. B. durch Umsetzung mit Komplexen von Schwefeltrioxid und einer Base, wie Pyridin, Trimethylamin, Picolin, Dimethylformamid usw. bei etwa 0-80 °C, in einem inerten organischen Lösungsmittel, z. B. in einem Aether wie Dioxan, in Pyridin, Dimethylformamid usw. In Stellung 3 kann, vorgängig oder nach letzterer Umsetzung, die Aminogruppe R¹ durch Abspaltung der Schutzgruppe freigesetzt werden ; z. B. können die Benzyloxycarbonyl- und die Benzhydrylgruppe hydrogenolytisch, z. B. durch Einwirken von Wasserstoff und Palladiumkohle, abgespalten werden, die t-Butoxycarbonylgruppe kann durch Behandeln mit Trifluoressigsäure oder Ameisensäure, die Trichloräthoxycarbonylgruppe wiederum durch Behandeln mit Zink und einer Protonensäure, wie Essigsäure oder Chlorwasserstoffsäure, abgespalten werden. Anschliessend kann die freigesetzte Aminogruppe R¹ mit einer entsprechend substituierten Carbonsäure bzw. einem reaktionsfähigen funktionellen Derivat davon (z. B. Säureanhydrid, Säureamid, aktivem Ester, z. B. einem Thioester, wie dem Benzthiazolylthioester) acyliert werden, wobei die verschiedensten Acylgruppen, wie sie z. B. aus der Penicillin- oder Cephalosporinchemie, bekannt sind, eingeführt werden können. Beispielsweise können in dieser Weise optisch einheitliche, antimikrobiell wirksame β-Lactame der allgemeinen Formel 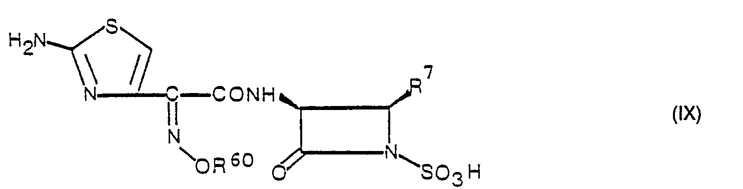 worin R⁶⁰ Wasserstoff, niederes Alkyl oder Carboxy-niederes Alkyl und R⁷ eine niedere organische Gruppe, z. B. Carbamoyl oder Carbamoyloxymethyl, darstellt, bzw. die entsprechenden optischen Antipoden davon nach an sich bekannten Methoden hergestellt werden.

Beispiele für entsprechende Ueberführungen in Endprodukte sind in den nachstehenden Reaktionsschemata I-III aufgeführt.

### (Siehe Schemata Seite 9 ff.)

### Erklärungen zu den Schemata I und II

DMB = 2,4-Dimethoxybenzyl (kann durch andere Schutzgruppen Z' ersetzt werden)
DMF = Dimethylformamid
PrOH = n-Propanol
DMSO = Dimethylsulfoxid
Py = Pyridin
Py · SO₃ = Schwefeltrioxid-pyridiniumkomplex
COX = reaktionsfähiges Derivat einer Carbonsäure, z. B. Säureanhydrid, Säureamid, aktiver Ester, z. B. Benzthiazolylester
R⁶ = Wasserstoff, niederes Alkyl (z. B. Methyl), geschütztes Carboxy-niederes Alkyl, z. B. geschütztes Carboxy-methyl, geschütztes 1-Methyl-1-carboxy-äthyl. Schutzgruppe : z. B. t-Butyl (mit z. B. Trifluoressigsäure abspaltbar), Benzyl und p-Nitrobenzyl (mit z. B. Wasserstoff und Palladiumkohle abspaltbar), 2-(Trimethylsilyl)-äthyl (mit z. B. Tetrabutylammoniumfluorid abspaltbar)
R⁶⁰ = Wasserstoff, niederes Alkyl (z. B. Methyl), Carboxy-niederes Alkyl, z. B. Carboxymethyl, 1-Methyl-1-carboxy-äthyl.

Die nachfolgenden Beispiele dienen lediglich der näheren Erläuterung der vorliegenden Erfindung, sollen dieselbe jedoch in keiner Weise einschränken. Alle Temperaturen sind in Celsiusgraden angegeben. Die optische Drehung [α]_{D} wurde bei 20 °C gemessen.

### Abkürzungen bei den Analysendaten :

Ft = Phthalimido ;
Ar = Aromat ;
Me = Methyl ; 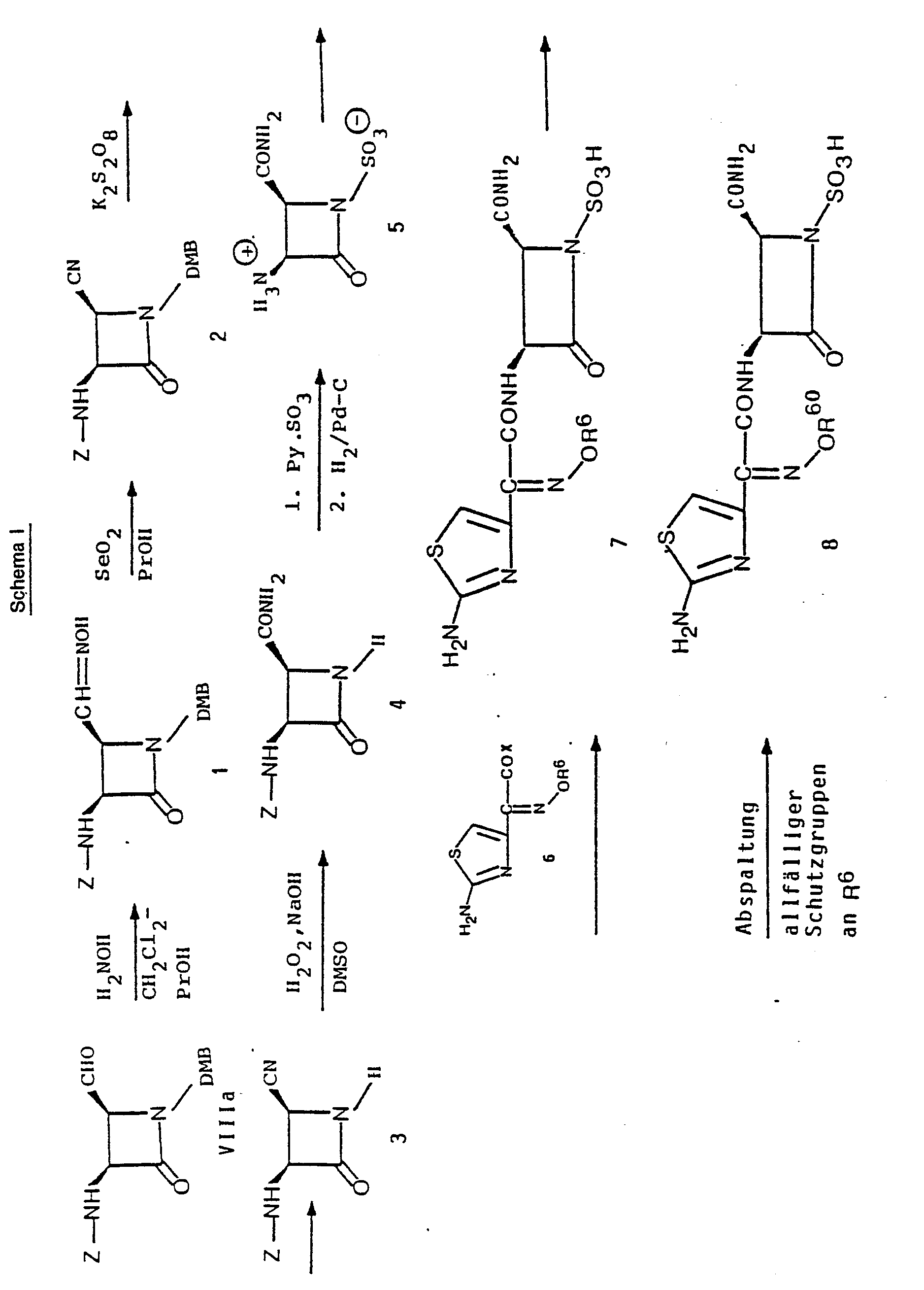 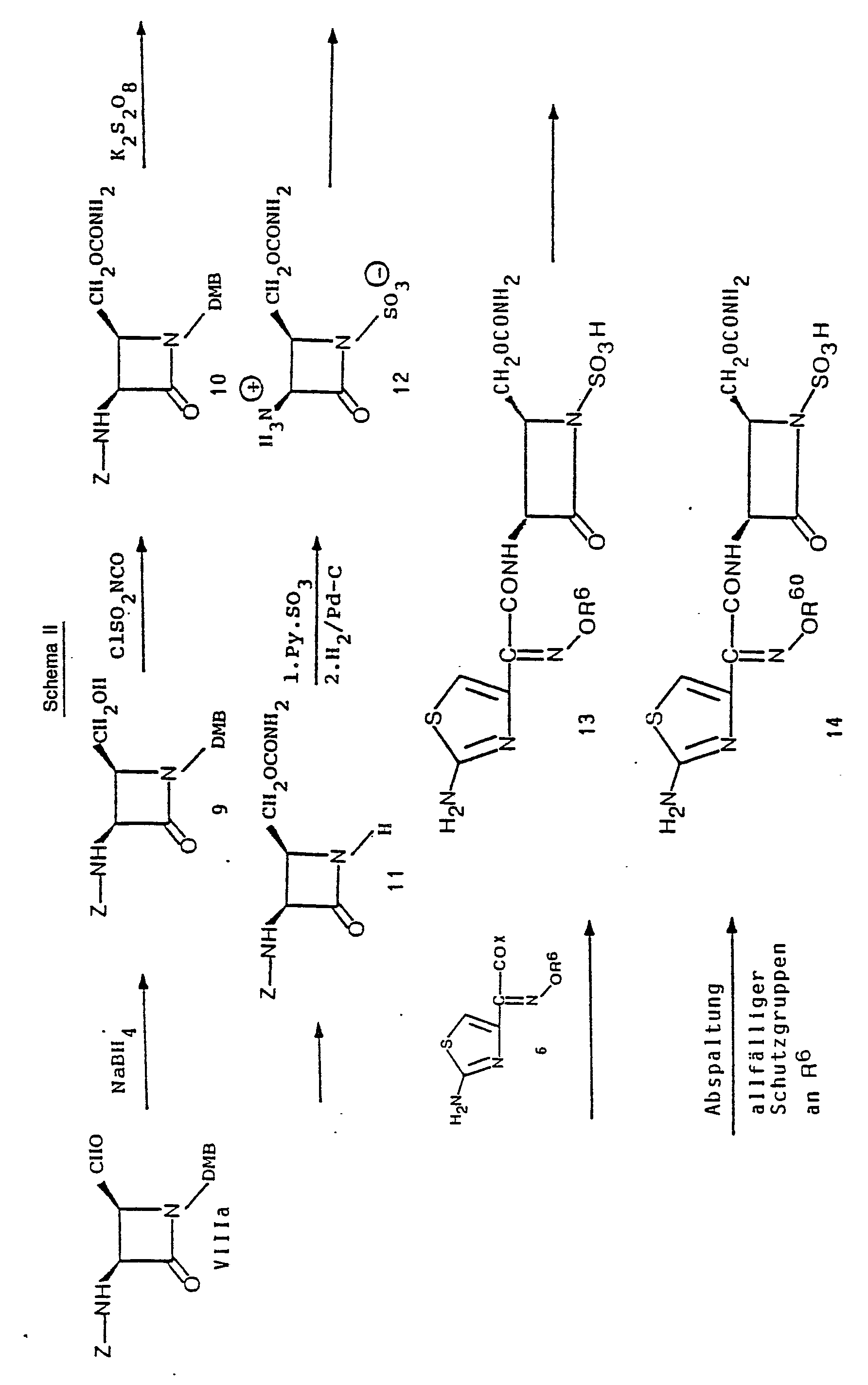 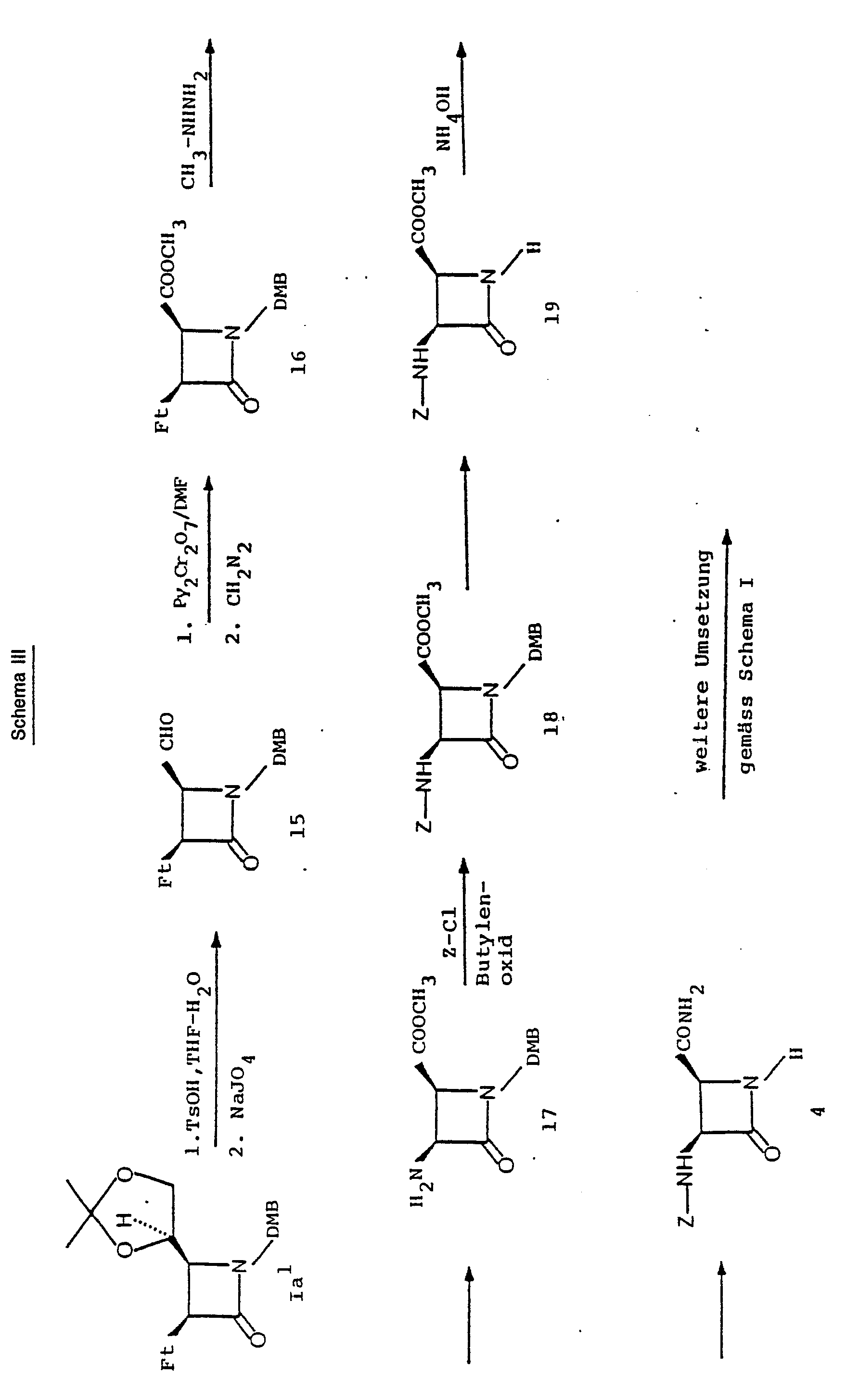
Ø = Phenyl ;
Bz = Benzyl ;

### Beispiel 1

Zu einer bei Raumtemperatur gerührten Lösung von 4,9 g (37,65 mMol) Isopropyliden-D-glyceraldehyd in 100 ml trockenem Methylenchlorid (frei von Methanol) gibt man 10 g Molekularsieb 4 Å und anschliessend tropfenweise eine Lösung von 6,29 g (37,65 mMol) 2,4-Dimethoxybenzylamin in 20 ml trockenem Methylenchlorid. Man rührt die Reaktionsmischung während 2 Stunden bei Raumtemperatur, versetzt anschliessend mit 5 g wasserfreiem Magnesiumsulfat, rührt noch während 30 Minuten und filtriert anschliessend, wobei der Filterkuchen noch mit 20 ml Methylenchlorid gewaschen wird.

Die erhaltene organische Lösung von Isopropyliden-D-glyceraldehyd-(2,4-dimethoxybenzyl)imin wird unter Argon auf -18° gekühlt und unter Rühren mit 5,5 ml (39,5 mMol) Triäthylamin versetzt. Nach wenigen Minuten gibt man über einen Zeitraum von 1 Stunde eine Lösung von 8,8 g (39,3 mMol) Phthaloylglycylchlorid in 100 ml trockenem Methylenchlorid dazu, rührt die Reaktionsmischung während 2 Stunden bei -15° und lässt anschliessend auf Raumtemperatur erwärmen. Man rührt noch über Nacht bei Raumtemperatur, wäscht die Reaktionsmischung dreimal mit je 100 ml Wasser und mit 100 ml Kochsalzlösung und trocknet die erhaltene Lösung über Natriumsulfat. Man dampft ein und chromatographiert den erhaltenen Rückstand an einer Kieselgelsäule (Korngrösse : 230-400 mesh) unter Eluieren mit Hexan/Essigester (1 : 1). Man erhält 15,8 g (90 %) N-[(3R,4R)-cis-1-(2,4-Dimethoxybenzyl)-4-[(S)-2,2-dimethyl-1,3-dioxolan-4-yl]-2-oxo-3-azetidinyl]phthalimid als Schaum ; [a]_{D} = -37° (c = 1 in Chloroform) ; MS : 466 (M⁺). Nach der Umkristallisation aus Aethylacetat erhält man farblose Kristalle vom Schmelzpunkt 155° ; [α]_{D} = ―49,2 (c = 0,8 in Chloroform).

### Beispiel 2

Man versetzt eine Lösung von 9,7 g (20,8 mMol) N-[(3R,4R)-cis-1-(2,4-Dimethoxybenzyl)-4-[(S)-2,2-dimethyl-1,3-dioxolan-4-yl]-2-oxo-3-azetidinyl]phthalimid in 120 ml Methylenchlorid mit 2,17 ml (40,8 mMol) Methylhydrazin. Man rührt über Nacht bei 28°, filtriert vom ausgefallenen Material ab und dampft das Filtrat unter vermindertem Druck ein. Man nimmt den Rückstand in 70 ml Essigester auf und filtriert die erhaltene Suspension. Das Filtrat wird dreimal mit je 100 ml Wasser und mit 150 ml Kochsalzlösung gewaschen und über Natriumsulfat getrocknet. Nach Eindampfen des Lösungsmittels erhält man 5,2 g (74 %) rohes (3R,4R)-cis-3-Amino-1-(2,4-dimethoxybenzyl)-4-[(S)-2,2-dimethyl-1,3-dioxolan-4-yl]-2-azeti- dinon, das ohne weitere Reinigung in die nächste Stufe eingesetzt wird.

### Beispiel 3

Man versetzt eine gerührte Lösung von 5,2 g (15,47 mMol) (3R,4R)-cis-3-Amino-1-(2,4-dimethoxybenzyl)-4-[(S)-2,2-dimethyl-1,3-dioxolan-4-yl]-2-azetidinon und 4,8 ml (62 mMol) Butylenoxid in 120 ml Methylenchlorid tropfenweise mit 2,8 ml (20 mMol) Carbobenzoxychlorid, rührt während 1 Stunde und dampft anschliessend unter vermindertem Druck ein. Das erhaltene rohe Material wird mit trockenem Aether behandelt, wobei man ein kristallines Material erhält, das man an einer Kieselgelsäule (Korngrösse : 230-400 mesh) unter Eluieren mit Hexan/Essigester (1 :1) chromatographiert. Man erhält 6,53 g (90 %) Benzyl-(3R,4R)-cis-1-(2,4-dimethoxybenzyl)-4-[(S)-2,2-dimethyl-1,3-dioxolan-4-yl]-2-oxo-3-azeti- dincarbamat vom Schmelzpunkt 115-116 ; [α]_{D} =-46° (c = 0,3 in Methanol).

### Beispiel 4

Man versetzt eine gerührte Lösung von 6,53 g (13,87 mMol) Benzyl-(3R,4R)-cis-1-(2,4-dimethoxybenzyl)-4-[(S)-2,2-dimethyl-1,3-dioxolan-4-yl]-2-oxo-3-azetidincarbamat in 450 ml Acetonitril bei 90° und unter Argon tropfenweise mit einer Lösung von 6,0 g (22,2 mMol) Kaliumperoxodisulfat und 3,75 g (21,55 mMol) Dikaliumhydrogenphosphat in 150 ml Wasser. Man rührt während 2 Stunden, lässt die Reaktionsmischung abkühlen und stellt den pH durch Zugabe von überschüssigem Dikaliumhydrogenphosphat (ca. 6 g) auf pH 6-7 ein. Man filtriert und dampft das Filtrat unter vermindertem Druck ein. Den Rückstand nimmt man in 200 ml Methylenchlorid auf, wäscht viermal mit je 70 ml Wasser und mit 70 ml Kochsalzlösung. Nach Trocknen und Eindampfen wird der ölige Rückstand an einer Kieselgelsäule (Korngrösse : 230-400 mesh) unter Eluieren mit Essigester/n-Hexan (7 : 3) chromatographiert. Man erhält 2,52 g (56,7 %) Benzyl-(3R,4R)-cis-4-[(S)-2,2-dimethyl-1,3-dioxolan-4-yl]-2-oxo-3-azetidincarbamat vom Schmelzpunkt 140-141 ° ; [α]_{D} = -46° (c = 0,5 in Methanol). Die absolute Konfiguration dieser Verbindung wurde durch Röntgenstrukturanalyse bestätigt.

### Beispiel 5

Eine Lösung von 320 mg (0,68 mMol) Benzyl-(3R,4R)-cis-1-(2,4-dimethoxybenzyl)-4-[(S)-2,2-dimethyl-1,3-dioxolan-4-yl]-2-oxo-3-azetidincarbamat in 40 ml Methanol wird in Gegenwart von 4 g Amberlite IR 120 (H⁺-Form) über Nacht gerührt. Die Reaktionsmischung wird filtriert, wobei man das polymere Material zweimal mit je 20 ml Methanol sorgfältig wäscht. Die vereinigten Filtrate werden eingedampft, und man erhält 272 mg (97,2 %) reines Benzyl-(3R,4R)-cis-4-[(S)-1,2-dihydroxyäthyl]-1-(2,4-dimethoxybenzyl)-2-oxo-3-azetidincarbamat.

### Beispiel 6

Man versetzt eine Lösung von 90 mg (0,21 mMol) Benzyl-(3R,4R)-cis-4-[(S)-1,2-dihydroxyäthyl]-1-(2,4-dimethoxybenzyl)-2-oxo-3-azetidincarbamat in 18 ml Methanol unter Rühren tropfenweise mit einer Lösung von 60 mg (0,28 mMol) Natriummetaperjodat in 2 ml Wasser. Man rührt während 1 Stunde, filtriert und dampft das Filtrat unter vermindertem Druck ein. Man nimmt den Rückstand in 20 ml Essigester auf und wäscht zweimal mit je 10 ml Wasser und mit 10 ml Kochsalzlösung. Nach Trocknen und Eindampfen erhält man 66 mg (76 %) reines Benzyl-(3R,4R)-cis-1-(2,4-dimethoxybenzyl)-4-formyl-2-oxo-3-azeti- dincarbamat vom Schmelzpunkt 147-149° (aus Essigester/Hexan) ; [α]_{D} = ―32° (c = 0,2 in Methanol).

### Beispiel 7

a) Zu einer bei Raumtemperatur gerührten Lösung von 0,9 g (5,4 mMol) 2,4-Dimethoxybenzylamin in 100 ml Methyllenchlorid gibt man 3 g Molekularsieb 4 A und nach 20 Minuten 0,7 g (5,4 mMol) Isopropyliden-L-glyceraldehyd und 5 wasserfreies Magnesiumsulfat. Anschliessend rührt man noch während 1 Stunde bei Raumtemperatur. Die erhaltene organische Lösung von Isopropyliden-L-glyceraldehyd-(2,4-dimethoxybenzyl)imin wird unter Argon auf -20° gekühlt und unter Rühren mit 0,88 ml (5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Man arbeitet auf wie in Beispiel 1 beschrieben und erhält nach der Chromatographie 1,77 g (70 %) N-[(3S,4S)-cis-1-(2,4-Dimethoxybenzyl)-4-[(R)-2,2-dimethyl-1,3-dioxolan-4-yl]-2-oxo-3-azetidinyl]-phthalimid als Schaum ; [α]_{D} = + 41° (c = 0,8 in Chloroform) ; MS : 466 (M⁺). Nach dem Umkristallisieren aus Aethylacetat erhält man ein kristallines Produkt mit [a]ₒ = + 48° (c = 0,6 in Chloroform).
b) Aus 1,6 g (3,4 mMol) N-[(3S,4S)-cis-1-(2,4-Dimethoxybenzyl)-4-[(R)-2,2-dimethyl-1,3-dioxolan-4-yl]-2-oxo-3-azetidinyl]-phthalimid erhält man in Analogie zu den Angaben in Beispiel 2 1,0 g (87 %) rohes (3S,4S)-cis-3-Amino-1-(2,4-dimethoxybenzyl)-4-[(R)-2,2-dimethyl-1,3-dioxolan-4-yl]-2-azetidinon.
c) Aus 1,0 g (3,0 mMol) (3S,4S)-cis-3-Amino-1-(2,4-dimethoxybenzyl)-4-[(R)-2,2-dimethyl-1,3-dioxolan-4-yl]-2-azetidinon erhält man in Analogie zu den Angaben in Beispiel 3 1,2 g (85 %) Benzyl-(3S,4S)-cis-1-(2,4-dimethoxybenzyl)-4-[(R)-2,2-dimethyl-1,3-dioxolan-4-yl]-2-oxo-3-azetidincarbamat vom Schmelzpunkt 115-117°.
d) Aus Benzyl-(3S,4S)-cis-1-(2,4-dimethoxybenryl)-4-[(R)-2,2-dimethyl-1,3-dioxolan-4-yl]-2-oxo-3-azetidin- carbamat kann man in Analogie zu den Angaben in Beispiel 4 Benzyl-(3S,4S)-cis-4-[(R)-2,2-dimethyl-1,3-dioxolan-4-yl]-2-oxo-3-azetidincarbamat herstellen.
e) Benzyl-(3S,4S)-cis-1-(2,4-dimethoxybenzyl)-4-[(R)-2,2-dimethyl-1,3-dioxolan-4-yl]-2-oxo-3-azeti- dincarbamat kann in Analogie zu den Angaben in Beispiel 5 in Benzyl-(3S,4S)-cis-4-[(R)-1,2-dihydroxy- äthyl]-1-(2,4-dimethoxybenzyl)-2-oxo-3-azetidincarbamat übergeführt werden.
f) Aus Benzyl-(3S,4S)-cis-4-[(R)-1,2-dihydroxyäthyl]-1-(2,4-dimethoxybenzyl)-2-oxo-3-azetidincarba- mat kann in Analogie zu den Angaben in Beispiel 6 Benzyl-(3S,4S)-cis-1-(2,4-dimethoxybenzyl)-4-formyl-2-oxo-3-azetidincarbamat erhalten werden.

### Beispiel 8

a) 7,0 g Benzyl-(3S,4S)-cis-1-(2,4-dimethoxybenzyl)-4-[(R)-2,2-dimethyl-1,3-dioxolan-4-yl]-2-oxo-3- azetidincarbamat (0,015 Mol) werden in 100 ml Acetonitril gelöst. Diese Lösung wird tropfenweise zu einer auf 98° gewärmten Lösung von 6,5 g Kaliumperoxodisulfat (0,024 Mol) und 4,2 g Dikaliumhydrogenphosphat (0,024 Mol) in 100 ml Wasser gegeben. Wahrend der Zugabe wird der pH-Wert durch Zusatz von weiteren 10 g Dikaliumhydrogenphosphat auf 6 gehalten. Das Reaktionsgemisch wird 14 Stunden gerührt, anschliessend abgekühlt, filtriert und teilweise eingedampft. Die wässrige Phase wird anschliessend mit Aethylacetat extrahiert. Die organische Phase wird nacheinander mit Wasser und wässriger Kochsalzlösung gewaschen, über Natriumsulfat getrocknet, filtriert und eingedampft. Das erhaltene Oel wird an Kieselgel chrommatographiert (230-400 mesh, n-Hexan/Aethylacetat 1 : als Eluierungsmittel). Man erhält 3,9 g (82 %) Benzyl-(3S,4S)-cis-4-[(R)-2,2-dimethyl-1,3-dioxolan-4-yl]-2-oxo-3-azetidincarbamat als farblose Kristalle vom Schmelzpunkt 142°. MS : 305 (M-CH₃) [a]_{D} = + 57° (c = 0,5 in Methanol).
b) 350 mg (1,09 mMol) Benzyl-(3S,4S)-cis-4-[(R)-2,2-dimethyl-1,3-dioxolan-4-yl]-2-oxo-3-azeti- dincarbamat werden in 25 ml Methanol gelöst und 12 Stunden bei Zimmertemperatur in Gegenwart von 10 g Amberlite IR 120 (H⁺⁻Form ; mit Methanol vorgewaschen) gerührt. Das Reaktionsgemisch wird anschliessend filtriert und mit einer wässrigen Lösung von 232 mg Natriummetaperiodat (1,08 mMol) tropfenweise behandelt. Nach 2-stündigem Rühren wird das Reaktionsgemisch teilweise eingedampft und mit Aethylacetat extrahiert. Die organische Phase wird nacheinander mit Wasser und wässriger Kochsalzlösung gewaschen, über Natriumsulfat getrocknet, filtriert und eingedampft. Man erhält 250 mg (92 %) Benzyl-(3S,4S)-cis-4-formyl-2-oxo-3-azetidincarbamat.
   MS : 248 (M⁺).

### Beispiel 9

1 g (1,2 : 3,4)-di-O-Isopropyliden-α-D-galacto-hexodialdo-1,5-pyranose (3,87 mMol) werden in 60 ml absolutem Methylenchlorid, enthaltend 5 g Molekularsieb 4 Å, unter Rühren in einer Argonatmosphäre gelöst. Die Lösung wird bei Zimmertemperatur mit einer Lösung von 0,68 g 2,4-Dimethoxybenzylamin (4 mMol) in 10 ml Methylenchlorid tropfenweise behandelt. Die Lösung wird bei Zimmertemperatur 30 Minuten gerührt, anschliessend auf ―20° abgekühlt und mit 0,65 ml (0,47 g, 4,6 mMol) Triäthylamin behandelt und nach einigen Minuten mit einer Lösung von 0,8 g (0,40 mMol) Phthaloylglycylchlorid in 20 ml Methylenchlorid. Das Reaktionsgemisch wird auf Zimmertemperatur erwärmt und anschliessend 1,5 Stunden gerührt. Nach Filtration wird die erhaltene organische Lösung mit Wasser (2 mal 150 ml) und wässriger Kochsalzlösung (1 mal 100 ml) gewaschen und über Magnesiumsulfat getrocknet. Nach Eindampfen des Lösungsmittels und chromatographischer Reinigung an Kieselgel (230-400 mesh ; n-Hexan : Aethylacetat 1 : 1 als Lösungsmittel) erhält man 1,8 g (75 %) N-[cis-1-(2,4-Dimethoxybenzyl)-2-oxo-4-[(3aαH,8bαH)-tetrahydro-2,2,7,7-tetramethyl-5H-bis[1,3]dioxolo[4,5-b : 4',5'-d]pyran-5-yl]-3-azeti- dinyl]-phthalimid als Schaum. [α]_{D} = ― 27° (c = 1 % in Methanol) MS : 594 (M⁺). IR 1 767, 1 722 cm⁻¹ (KBr).

### Beispiel 10.

2,0 g (11,76 mMol) Cyclohexyliden-D-glyceraldehyd werden in 8 ml absolutem Methylenchlorid gelöst und bei Zimmertemperatur mit 2,06 g (12,35 mMol) 2,4-Dimethoxybenzylamin behandelt. Nach 30- minütigem Rühren wird das Reaktionsgemisch auf 0° abgekühlt und mit 1,96 ml (14,11 mMol) Triäthylamin und tropfenweise mit einer Lösung von 2,76 g (12,35 mMol) Phthaloylglycylchlorid in 4 ml Methylenchlorid behandelt. Das Reaktionsgemisch wird 12 Stunden bei Zimmertemperatur gerührt, anschliessend mit 100 ml Methylenchlorid verdünnt und nacheinander mit 30 ml Wasser, 30 ml 0,1 N wässriger Salzsäure, 30 ml Wasser und 30 ml wässriger Kochsalzlösung gewaschen und über Natriumsulfat getrocknet. Das Lösungsmittel wird abgedampft und der Rückstand an Kieselgel chromatographisch gereinigt (230-400 mesh ; Aethylacetat : n-Hexan 1 : 1 als Lösungsmittel). Man erhält 4,3 g (72 %) N-[(3R,4R)-1-(2,4-Dimethoxybenzyl)-4-[(S)-1,4-dioxaspiro[4.5]dec-2-yl]-2-oxo-3-azetidinyl]-phthali- mid, [α]_{D} = -18° (C = 0,5 in Methanol).
Elementaranalyse berechnet für 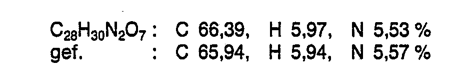
IR (KBr) cm⁻¹ : 3435, 1 768, 1 721, 1 589, 1 508, 1 208.
NMR(CDCl₃)δ(ppm) : 1,2-1,6 (10H, m, (CH₂)₅), 3,35 (1H, dd, 6 und 9 Hz, H-4), 3,5-3,8 (2H, m, O―CH₂) 3,81 und 3,82 (6H, 2s, 2 x OCH₃), 4,20 (1H, m, O-CH), 4,15 und 4,90 (2H, 2d, 14 Hz, N―CH₂―Ar), 5,25 (1 H, d, 6 Hz, H₃), 6,50 (2H, m, 2H Ar), 7,27 (1 H, d, Ar), 7,85 (4H, m, Ar).
MS : M⁺ : 506.

### Beispiel 11

Zu einer bei Raumtemperatur gerührten Lösung von 0,9 g (5,4 mMol) 3,4-Dimethoxybenzylamin in 100 ml Methylenchlorid gibt man 3g Molekularsieb 4 Å und nach 20 Minuten 0,7g (5,4 mMol) Isopropyliden-L-glyceraldehyd und 5 g wasserfreies Magnesiumsulfat. Anschliessend rührt man noch während 1 Stunde bei Raumtemperatur. Die erhaltene organische Lösung von Isopropyliden-L-glyceraldehyd-(3,4-dimethoxybenzyl)imin wird unter Argon auf -20° gekühlt und unter Rühren mit 0,88 ml (5,4 mMol) Triäthylamin versetzt. Dann wird innerhalb 1 Stunde eine Lösung von 1,25 g (5,6 mMol) Phthaloylglycylchlorid in 20 ml trockenem Methylenchlorid zugetropft und anschliessend über Nacht bei Raumtemperatur weiter gerührt. Man arbeitet auf wie in Beispiel 1 beschrieben und erhält nach der Chromatographie 1,56 (62 %) N-(3S,4S)-cis-1-(3,4-Dimethoxybenzyl)-4-[(R)-2,2-dimethyl-1,3-dioxolan-4-yl]-2-oxo-3-azetidinyl]phthalimid als Schaum ; [α]_{D} = + 16,2° (c = 1,0 in Methanol) ;
MS : 466 (M⁺).
Elementaranalyse: berechnet für 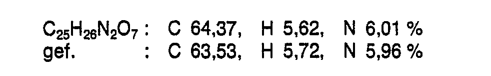
IR (KBr cm⁻¹) : 3 430, 1 766, 1 721, 1 609, 1 517

### Beispiel 12

Zu einer bei Raumtemperatur gerührten Lösung von 670 mg (4 mMol) 2,4-Dimethoxybenzylamin in 70 ml Methylenchlorid gibt man 2,5 g Molekularsieb 4 Ä und nach 20 Minuten 660 mg (4 mMol) (S)-2-Benzyloxypropionaldehyd und 3,7 g wasserfreies Magnesiumsulfat. Anschliessend rührt man 1 Stunde bei Raumtemperatur. Die erhaltene Lösung wird in einer Argonatmosphäre auf -20° gekühlt und unter Rühren mit 0,65 ml (4 mMol) Triäthylamin versetzt. Dann wird innerhalb 1 Stunde eine Lösung von 930 mg (4,15 mMol) Phthaloylglycylchlorid in 15 ml trockenem Methylenchlorid zugetropft und anschliessend 12 Stunden bei Raumtemperatur weiter gerührt. Man arbeitet auf wie in Beispiel 1 beschrieben und erhält nach der Chromatographie 990 g (50 %) N-[(3S,4S)-4-[(S)-1-(Benzyloxy)äthyl]-1-(2,4-dimethoxybenzyl)-2-oxo-3-azetidinyl]phthalimid als Schaum.
[α]_{D} = + 44.6 (c = 1 in Chloroform)
MS : 500 (M⁺).
Elementaranalyse : berechnet für

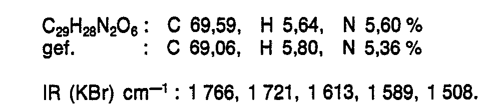

### Beispiel 13

20,8 g (44,59 mMol) N-[(3S,4S)-cis-1-(2,4-Dimethoxybenzyl)-4[(R)-2,2-dimethyl-1,3-dioxolan-4-yl]-2-oxo-3-azetidinyl]phthalimid werden in 300 ml Tetrahydrofuran : Wasser (2 : 1) gelöst und mit 1,5 g (7,88 mMol) p-Toluolsulfonsäuremonohydrat behandelt. Das Reaktionsgemisch wird 48 Stunden leise unter Rückflussbedingungen erhitzt und anschliessend abgekühlt. Der pH-Wert wird durch Zugabe von gesättigter wässriger Natriumbicarbonatlösung auf 7 eingestellt und die erhaltene Lösung unter Rühren mit einer wässrigen Lösung von 10,5 g (49,11 mMol) Natriummetaperjodat behandelt. Nach einer Stunde wird das Reaktionsgemisch filtriert und die Mutterlauge unter vermindertem Druck teilweise eingedampft. Die erhaltene wässrige Lösung wird zweimal mit 200 ml Aethylacetat extrahiert. Die organische Phase wird nacheinander mit 200 ml Wasser und 150 ml wässriger Kochsalzlösung gewaschen und über Natriumsulfat getrocknet. Nach dem Eindampfen und chromatographischer Reinigung (230-400 mesh ; Aethylacetat: n-Hexan 7 : 3 als Eluierungsmittel) erhält man N-[(3S,4S)-cis-1-(2,4-Dimethoxybenzyl)-4- formyl-2-oxo-3-azetidinyl]phthalimid in einer Ausbeute von 15,96 g (90,8 %).
NMR (CDCl₃)δ(ppm) : 3,80 (6H, s, 2 x OCH₃), 4,10 (1 H, dd, 3 und 5 Hz, CH―CHO) 4,55 (2H, s, N―CH₂) 5,50 (1 H, d, 5 Hz, Ft―CH), 6,40 (2H, m, Ar) 7,20 (1 H, m, Ar), 7,70 (4H, s, Ar), 9,50 (1 H, d, 3 Hz, CHO).

### Beispiel 14

a) 9,8 g (26 mMol) Pyridiniumdichromat werden in 20 ml absolutem Dimethylformamid gelöst und bei Zimmertemperatur mit einer Lösung von 5,1 g (13 mMol) N-[(3S,4S)-cis-1-(2,4-Dimethoxybenzyl)-4- formyl-2-oxo-3-azetidinyl]-phthalimid in 20 ml absolutem Dimethylformamid behandelt. Das Reaktionsgemisch wird 12 Stunden bei Zimmertemperatur gerührt, anschliessend in Eiswasser gegossen und das Ganze zweimal mit je 100 ml Aethylacetat extrahiert. Die vereinigten organischen Phasen werden nacheinander zweimal mit 100 ml Wasser, zweimal mit 100 ml wässriger Kochsalzlösung gewaschen und über Natriumsulfat getrocknet. Nach Abdampfen des Lösungsmittels erhält man 2,5 g (48 %) (3S,4S)-cis-1-(2,4-Dimethoxybenzyl)-3-phthalimido-2-azetidinon-4-carbonsäure als Schaum.
   MS : 410 (M⁺), 263
   IR (KBr, cm⁻¹) : 2500, 1 767, 1 724, 1 612, 1 509
   NMR (DMSO-d₆)δ(ppm) : 4,85 (6H, s, 2 x OCH₃), 4,20 (1H, d, 5,5 Hz, CH―COOH) 4,40 und 4,90 (2H, 2d, 14 Hz, N-CH₂), 5,55 (1 H, d, 5,5 Hz, Ft-CH), 6,50 (2H, m, Ar), 7,30 (1H, m, Ar), 7,80 (4H, m, Ar)
b) Eine Suspension von 250 mg (0,61 mMol) (3S,4S)-cis-1-(2,4-Dimethoxybenzyl)-3-phthalimido-2- azetidinon-4-carbonsäure in absoluten Aether wird mit 20 ml einer ätherischen Lösung von Diazomethan bis zum Abklingen der Stickstoffgasentwicklung behandelt. Das Reaktionsgemisch wird eingedampft und an Kieselgel chromatographiert (230-400 mesh, Aethylacetat : n-Hexan 1 : 1 als Eluierungsmittel). Man erhält 200 mg (77%) (3S,4S)-cis-1-(2,4-Dimethoxybenzyl)-3-phthalimido-2-azetidinon-4-carbonsäuremethylester.
   NMR (CDCl₃)δ(ppm) : 3,63 (3H, s, COOCH₃), 3,81 (6H, s, 2 x OMe), 4,20 (1 H, d, 5,5 Hz CHCOOMe), 4,40 und 4,90 (2H, 2d, 14 Hz, N―CH₂), 5,49 (1 H, d, 5,5 Hz, Ft―CH), 6,50 (2H, m, Ar) 7,20 (1 H, m; Ar), 7,90 (4H, m, Ar).

### Beispiel 15

a) Zu einer bei Raumtemperatur gerührten Lösung von 0,9 g (5,4 mMol) 2,4-Dimethoxybenzylamin in 100 ml Methylenchlorid gibt man 3 g Molekularsieb 4 Å und nach 20 Minuten 0,7 (5,4 mMol) Isopropyliden-L-glyceraldehyd und 5 wasserfreies Magnesiumsulfat. Anschliessend rührt man noch während 1 Stunde bei Raumtemperatur. Die erhaltene organische Lösung von Isopropyliden-L-glyceraldehyd-(2,4-dimethoxybenzyl)imin wird unter Argon auf -20° gekühlt und unter Rühren mit 0,88 ml (5,4 mMol) Triäthylamin versetzt. Dann wird innerhalb 1 Stunde eine Lösung von 1,25 (5,6 mMol) Phthaloylglycylchlorid in 20 ml trockenem Methylenchlorid zugetropft und anschliessend über Nacht bei Raumtemperatur weiter gerührt. Die Reaktionsmischung wird dreimal mit je 100 ml Wasser und mit 100 ml Kochsalzlösung gewaschen und über Natriumsulfat getrocknet. Man dampft ein und chromatographiert den Rückstand an Kieselgel (230-400 mesh) unter Eluieren mit Hexan/Essigester (1 : 1). Man erhält 1,77 g (70 %) N-[(3S,4S)-cis-1-(2,4-Dimethoxybenzyl)-4-[(R)-2,2-dimethyl-1,3-dioxolan-4-yl]-2-oxo-3- azetidinyl]-phthalimid als Schaum ; [α]_{D} = + 41° (c = 0,8 in Chloroform) ; MS : 466 (M⁺).
b) Man versetzt eine Lösung von 149,3 g (0,32 Mol) N-[(3S,4S)-cis-1-(2,4-Dimethoxybenzyl)-4-[(R)-2,2-dimethyl-1,3-dioxolan-4-yl]-2-oxo-3-azetidinyl]phthalimid in 2,5 I Methylenchlorid mit 34 ml (0,64 Mol) Methylhydrazin. Man rührt über Nacht bei 28°, filtriert vom ausgefallenen Material ab und dampft das Filtrat unter vermindertem Druck ein. Man nimmt den Rückstand in 1,2 I Essigester auf und filtriert die erhaltene Suspension. Das Filtrat wird dreimal mit je 500 ml Wasser und mit 500 ml Kochsalzlösung gewaschen und über Natriumsulfat getrocknet. Nach Eindampfen des Lösungsmittels erhält man 104,3 g (86,8 %) rohes (3S,4S)-cis-3-Amino-1-(2,4-dimethoxybenzyl)-4-[(R)-2,2-dimethyl-1,3-dioxolan-4-yl]-2-azeti- dinon, das ohne weitere Reinigung in die nächste Stufe eingesetzt wird.
c) Man versetzt eine gerührte Lösung von 104 (0,31 Mol) (3S,4S)-cis-3-Amino-1-(2,4-dimethoxybenzyl)-4-[(R)-2,2-dimethyl-1,3-dioxolan-4-yl]-2-azetidinon und 104 ml (1,2 Mol) Butylenoxid in 1,5 I Methylenchlorid tropfenweise mit 57,6 ml (0,4 Mol) Carbobenzoxychlorid, rührt während 1 Stunde und dampft anschliessend unter vermindertem Druck ein. Das erhaltene rohe Material wird mit 2 I trockenem Aether behandelt, wobei man ein kristallines Material erhält. Man erhält 122,6 g (84 %) Benzyl-(3S,4S)-cis-1-(2,4-dimethoxybenzyl)-4-[(R)-2,2-dimethyl-1,3-dioxolan-4-yl]-2-oxo-3-azetidincarbamat vom Schmelzpunkt 115-116° ; [a]_{D} = + 48° (c = 0,3 in Methanol).
d) Eine Lösung von 160 g (0,34 Mol) Benzyl-(3S,4S)-cis-1-(2,4-dimethoxybenzyl)-4-[(R)-2,2-dimethyl-1,3-dioxolan-4-yl]-2-oxo-3-azetidincarbamat in 1 000 ml Tetrahydrofuran und 400 ml Wasser wird in Gegenwart von 8 g p-Toluolsulfonsäure um 60° über Nacht gerührt. Die Reaktionsmischung wird mit ges. NaHCO₃ neutralisiert und Tetrahydrofuran wird eingedampft. Die wässrige Lösung wird dann mit 21 Essigester extrahiert. Nach Trocknung über Na₂S0₄ und Eindampfen erhält man 142 g (97,2 %) reines Benzyl-(3S,4S)-cis-4-[(R)1,2-dihydroxyäthyl]-1-(2,4-dimethoxybenzyl)-2-oxo-3-azetidincarbamat, Smp. 177-178° (MeOH).
e) Man versetzt eine Lösung von 142 g (0,33 Mol) Benzyl-(3S,4S)-cis-4-[(R)-1,2-dihydroxyäthyl]-1-(2,4-dimethoxybenzyl)-2-oxo-3-azetidincarbamat in 1 000 ml Tetrahydrofuran unter Rühren tropfenweise mit einer Lösung von 76,8 g (0,359 Mol) Natriummetaperjodat in 600 ml Wasser. Man rührt während 1 Stunde, filtriert und dampft das Filtrat unter vermindertem Druck ein. Man nimmt den Rückstand in 400 ml Essigester auf und wäscht zweimal mit je 100 ml Wasser und mit 50 ml Kochsalzlösung. Nach Trocknen und Eindampfen erhält man 105 g (87,8 %) reines Benzyl-(3S,4S)-cis-1-(2,4-dimethoxybenzyl)-4- formyl-2-oxo-3-azetidin-carbamat vom Schmelzpunkt 145-147° (aus Essigester/Hexan) ; [α]_{D} = + 13,7° (c = 1, CHCI₃).
f) 4,27 g (113 mMol) Natriumborhydrid werden in 1,6 I abs. Aethanol gelöst und auf 0° abgekühlt. Diese Lösung wird mit einer Lösung von 90 g (226 mMol) Benzyl-(3S,4S)-cis-1-(2,4-dimethoxybenzyl)-4- formyl-2-oxo-3-azetidincarbamat in 720 ml Aethanol-Tetrahydrofuran (1 : 1) tropfenweise versetzt. Das Reaktionsgemisch wird 2 Stunden bei 0° gerührt und anschliessend mit 350 ml gesättigter wässriger Natriumsulfatlösung behandelt und 45 Minuten gerührt. Nach dem Filtrieren und Abdampfen des Lösungsmittels wird der Rückstand in 1,5 I Aethylacetat aufgenommen und bis zu neutraler Reaktion gewaschen. Nach dem Trocknen über Natriumsulfat und teilweisem Eindampfen erhält man kristallines Benzyl-(3S,4S)-cis-1-(2,4-dimethoxybenzyl)-4-hydroxymethyl-2-oxo-3-azetidincarbamat in Form von 72,2 g farblosen Kristallen (79,6 %) vom Schmelzpunkt 138°, [α]_{D} = + 41,6° (c = 1 in Methanol).
   Elementaranalyse : berechnet für C₂₁H₂₄N₂O₆ : 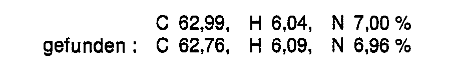
   IR (KBr) cm⁻¹ : 1 718, 1 698, 1 615, 1 589
   NMR (CDCl₃)δ(ppm) : 2,45 (1H,dd,OH), 3,55-3,75 (3H, breit, CH―CH₂―), 3,79 (6H,s,2 x OCH₃), 4,35 (2H,s,N-CH₂), 5,08 (2H,s,0-CH₂), 5,02-5,18 (1 H,dd,5 und 9 Hz, H-3), 6,06 (1 H,d,9Hz,NH), 6,43 (2H,m,Ar), 7,15 (1 H,m,Ar), 7,31 (5H,m,C₆H₅)
   MS : 292 (M-BzOH)
g) Eine Lösung von 30 g (74,9 mMol) Benzyl-(3S,4S)-cis-1-(2,4-Dimethoxybenzyl)-4-hydroxymethyl-2-oxo-3-azetidincarbamat in 600 ml Methylenchlorid werden bei 0-5° mit 21,22 g Chlorsulfonyl-isocyanat (2 Aequivalenten) behandelt. Nach 15 Minuten wird das Reaktionsgemisch tropfenweise zu einer wässrigen, auf 5° gekühlten Lösung von 20,9 g (2,7 Aequivalenten) Natriumsulfit gegeben. Das Reaktionsgemisch wird 2 Stunden gerührt, anschliessend mit Methylenchlorid verdünnt und die organische Phase abgetrennt, mit wässriger Natriumchloridlösung gewaschen und 12 Stunden über Natriumsulfat getrocknet. Die organische Phase wird anschliessend mit Magnesiumsulfat behandelt und weitere 2 Stunden gerührt. Nach dem Filtrieren und Abdampfen des Lösungsmittels wird der Rückstand mit Aether behandelt, die erhaltenen Kristalle werden filtriert und mit Aether gewaschen. Man erhält 32,6 g (97 %) (3S,4S)-cis-3-Benzyloxycarboxamido-4-carbamoyloxymethyl-1-(2,4-dimethoxybenzyl)-2-azetidinon vom Schmelzpunkt 178-179°, [a]_{D} = + 84,7° (C = 0,8 in Chloroform).
   Elementaranalyse : berechnet für C₂₂H₂₅N₃O₇ : 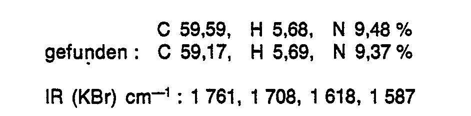
h) Eine Suspension von 11,9 g (26,8 mMol) (3S,4S)-cis-3-Benzyloxycarboxamido-4-carbamoyloxymethyl-1-(2,4-dimethoxybenzyl)-2-azetidinon, 14,5 g (53,5 mMol) Kaliumperoxodisulfat, 13,98 g (80,3 mMol) Dikaliumhydrogenphosphat und 1,33 g (5,36 mMol) Kupfersulfat (5H₂0) in 270 ml Acetonitril und 130 ml Wasser werden in einer Argonatmosphäre 3 1/2 Stunden auf 95° erhitzt, bei einem pH-Wert zwischen 6,5 und 7,0 (zeitweilige Zugabe von 10 g Dikaliumhydrogenphosphat). Nach dem Erkalten und Filtrieren wird die wässrige Phase verworfen und die organische Phase eingedampft. Der Rückstand wird in Aethylacetat aufgenommen und mit Wasser und Kochsalzlösung gewaschen. Nach dem Trocknen über Natriumsulfat, Filtration und Abdampfen des Lösungsmittels wird der Rückstand in Aether aufgenommen und filtriert. Die rohen Kristalle (8,9 g) werden auf SiO₂ chromatographiert (300 g, 40-63 µm, Chloroform : Methanol : Aethylacetat 85 :10 :5). Man erhält 5,5 g (70 %) (3S,4S)-cis-3-Benzyloxycarboxamido-4-carbamoyloxymethyl-2-azetidinon als farblose Kristalle, [α]_{D} = + 61,2° (c = 1 in Methanol). Schmelzpunkt 193-195°.
   Elementaranalyse : berechnet für C₁₃H₁₅N₃O₅ : 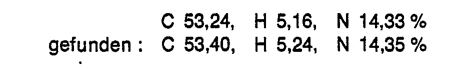
   IR (KBr) cm⁻¹ : 3 414, 3 315, 1 757, 1 701, 1 610, 1 540, 1 498
   NMR (d₆DMSO)δ(ppm): 3,31-4,06 (3H,m,CH-CH₂-), 4,95 (lH,dd,4,5 und 9Hz,H-3), 5,06 (2H,s,∅―CH₂), 6,53 (2H,breit,NH₂), 7,35 (5H,s,C₆H₅), 7,95 (1H,d,9Hz,C―3 NH―CO), 8,35 (1H,s,NH―CO)
   MS (CI mit NH₃) : 251 (M + H)+-CONH
i) 5,4 g (18.4 mMol) (3S,4S)cis-3-Benzyloxycarboxamido-4-carbamoyloxymethyl-2-azetidinon in 200 ml abs. Dioxan werden bei Zimmertemperatur mit 4,3 g (1,3 Aequivalenten) Pyridin-Schwefeltrioxid-Komplex behandelt. Die erhaltene Suspension wird 3 Stunden gerührt, anschliessend mit weiteren 0,99 g (0,3 Aequivalenten) Pyridin-Schwefeltrioxid-Komplex behandelt und das Reaktionsgemisch eine weitere Stunde gerührt. Nach Zugabe weiterer 1,37 g (0,4 Aequivalenten) Pyridin-Schwefeltrioxid-Komplex und weiterem 2-stündigen Rühren wird das Lösungsmittel teilweise unter vermindertem Druck entfernt und der Rückstand mit 110 ml gesättigter wässriger Natriumbicarbonatlösung behandelt. Die erhaltene, braune Lösung wird 12 Stunden im Kühlschrank stehen gelassen und die erhaltenen Kristalle werden abfiltriert. Die Mutterlauge wird chromatographiert (MCI Gel, Wasser-Aethanol 1 : 0 bis 9 : 1). Nach dem Lyophilisieren erhält man 3,5 g (49 %) (3S,4S)-cis-3-Benzyloxycarboxamido-4-carbamoyloxymethyl-2- azetidinon-1-sulfonsäure-Natriumsalz als ein farbloses Pulver, [α]_{D} = + 29,6° (c = 0,5 in Wasser).
   Elementaranalyse : berechnet für C₁₃H₁₄N₃O₈SNa : 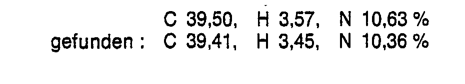
   IR (KBr) cm⁻¹ : 1 798, 1 758, 1 739, 1 693, 1 584, 1 547
   NMR (d₆DMSO)8(ppm): 3,9-4,4 (3H,m,CH―CH₂), 4,9 (dd,1H,NH―CH), 5,1 (s,2H,Ø-CH2), 6,4 (2H,breit,NH₂), 7,4 (5H,s,C₆H₅), 8,0 (1H,d,NH)
k) 3,065 g (7,75 mMol) (3S,4S)-cis-Benzyloxycarboxamido-4-carbamoyloxymethyl-2-azetidinon-1-sulfonsäure-Natriumsalz werden in 180 ml abs. Methanol gelöst und 1 Stunde in Gegenwart von 1,5 g 10 % Palladiumkohle hydriert. Der Katalysator wird durch Filtration entfernt und die erhaltene Lösung eingedampft. Man erhält 2,02 g (100%) (3S,4S)-cis-3-Amino-4-carbamoyloxymethyl-2-oxo-1-azetidin- sulfonsäure-Natriumsalz.
   IR (KBr) cm-¹ : 3 444, 3 207, 1 754, 1 725, 1 611, 1 249
I) 1,62 g (6,20 mMol) (3S,4S)-3-Amino-4-carbamoyloxymethyl-2-oxo-1-azetidinsulfonsäure-Natriumsalz werden in 180 ml Aceton-Wasser (2 : 1) gelöst und mit 3,27 g (6,83 mMol) (Z)-2-(2-Amino-4-thiazolyl)-2-[[1-[t-butoxycarbonyl]-1-methyläthoxy]imino]-essigsäure-2-benzthiazolyl-thioester behandelt. Das Reaktionsgemisch wird 12 Stunden bei Zimmertemperatur gerührt. Weitere 60 mg (0,12 mMol) von letzterer Verbindung werden hinzugefügt und das Rühren wird weitere 3 Stunden fortgesetzt. Aceton wird unter vermindertem Druck entfernt und 50 ml Wasser hinzugefügt. Die erhaltenen Kristalle werden abfiltriert und mit etwas Wasser gewaschen. Die Mutterlauge wird teilweise eingedampft (37°, 15 mmHg) und chromatographiert (MCI Gel, H₂0). Nach dem Lyophilisieren erhält man 2,28 g (77 %) (3S,4S)-3-[2-Amino-4-thiazolyl)-2-(Z)-[[1-(t-butoxycarbonyl)-1-methyläthoxy]imino]-acetamido]-4-carbamoyloxymethyl-2-oxo-1-azetidinsulfonsäure-Natriumsalz.
   IR (KBr) cm¹ : 1 766, 1 723, 1 683, 1 617, 1 531, 1 458, 1 369
   NMR (d₆DMSO)δ(ppm): 1,35 (15H,s,5 x CH₃), 4,0-4,15 (3H,m,H-4 und CH₂―OCONH₂), 5,25 (lH,dd,H-3), 6,5 (2H,breit,CONH₂), 6,7 (1H,s,H-Thiazol), 7,25 (2H,s,NH₂), 8,9 (1H,d,CO―NH).
m) 2,28 g (3,98 mMol) (3S,4S)-3-[2-Amino-4-thiazolyl)-2-(Z)-[[1-(t-butoxycarbonyl)-1-methyläthoxy]i-mino]acetamido]-4-carbamoyloxymethyl-2-oxo-1-azetidinsulfonsäure-Natriumsalz werden unter Eiskühlung mit 5 ml Trifluoressigsäure behandelt. Das Eisbad wird entfernt und das Reaktionsgemisch bei Zimmertemperatur 30 Minuten gerührt. Ueberschüssige Trifluoressigsäure wird unter vermindertem Druck (20 °C, 15 mmHg) entfernt und das erhaltene Oel mit 100 ml Aether behandelt. Die erhaltenen Kristalle werden abfiltriert, mit Aether gewaschen und unter stark vermindertem Druck getrocknet. Nach wässriger Reverse phase-Chromatographie erhält man nach dem Lyophilisieren 1,51 g (76,6 %) (3S,4S)-3-[(Z)-2-(2-Amino-4-thiazolyl)-2-[[1-carboxy-1-methyläthoxyl]imino]acetamido-4-carbamoyloxymethyl-2-oxo-1-azetidin-sulfonsäure, [a]_{D} = + 35,7° (c = 0,3 in Wasser).
   Elementaranalyse : berechnet für C₁₄H₁₈N₆O₁₀S₂ : 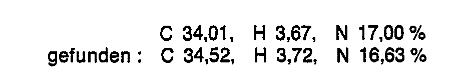
   IR (KBr) cm-¹ : 1 764, 1 722, 1 680, 1 637
   NMR (dₑDMSO)8(ppm) : 1,50 (6H,s,2 x CH₃), 4,00-4,20 (3H,CH―CH₂), 5,35 (1H,dd,4,5 und 9Hz,H―3), 6,50 (3H, breit, NH₃⁺ oder COOH, CONH₂), 6,90 (1 H,s,Thiazol-5H), 9,15 (1 H,d,9Hz,CONH).

Der als Ausgangsverbindung in Absatz 1) eingesetzte 2-(2-Amino-4-thiazolyl)-2-[[(Z)-1-(t-butoxycarbonyl)-1-methyl-äthoxy]-imino]-essigsäure-1-benzthiazolyl-thioester kann wie folgt hergestellt werden :
n) 43 g (200 mMol) 2-(2-Amino-4-thiazolyl-2-(Z)-hydroxyimino-essigsäure-äthylester werden in 1,2 I Dimethylformamid gelöst. In einer Stickstoffatmosphäre werden allmählich 89,2 g (400 mMol) 2-Brom-2-methyl-propionsäure-t-butylester hinzugefügt, gefolgt von 110,6 g (800 mMol) fein pulverisiertem Kaliumcarbonat. Das Reaktionsgemisch wird 12 Stunden bei 45° gerührt. Nach dem Erkalten bei Zimmertemperatur werden 4 I Wasser hinzugefügt, und das Ganze wird mit 3,5 I Aethylacetat extrahiert. Die organische Phase wird 3 mal mit 2 1 Wasser gewaschen. Das Wasser wird nochmals mit 1,5 I Aethylacetat extrahiert. Die vereinigten Aethylacetatlösungen werden mit Magnesiumsulfat getrocknet und zur Trockene eingedampft. Nach dem Umkristallisieren aus Aether erhält man 61,4 g (85,9 %) 2-(2-Amino-4-thiazolyl)-2-[[(Z)-1-(t-butoxycarbonyl)-1-methyläthoxylimino]-essigsäure-äthylester vom Schmelzpunkt 172°.
o) 240 g (671,5 mMol) 2-(2-Amino-4-thiazolyl-2-[[(Z)-1-(t-butoxycarbonyl)-1-methyl-äthoxyl]imino]-essigsäure-äthylester werden in 1,3 I Methanol und 1,34 I 1N wässriger Natriumlauge 12 Stunden bei 50° gerührt. Das Methanol wird abgedampft und die wässrige Phase 2 mal mit 1 I Aethylacetat gewaschen. Nach Zugabe von 1,34 I 1N wässriger Salzsäure kristallisiert das Produkt aus. Nach dem Erkalten auf 0° werden die Kristalle abfiltriert, nacheinander mit Wasser, Acetonitril und Aether gewaschen und unter vermindertem Druck bei 40° getrocknet. Das so erhaltene Produkt kristallisiert mit 12 % Wasser und wird zur Entfernung des Wassers 2 Stunden in Acetonitril gerührt. Nach dem Filtrieren und Trocknen unter vermindertem Druck bei 40° erhält man 177,7 g (80,3 %) 2-(2-Amino-4-thiazolyl)-2-[[(Z)-1-(t-butoxycarbonyl)-1-methyläthoxy]imino]-essigsäure vom Schmelzpunkt 178-179°. Wassergehalt 0,4 %.
p) 28,8 g (86,4 mMol) 2-(2-Amino-4-thiazolyl)-2-[[(Z)-1-(t-butoxycarbonyl)-1-methyl-äthoxy]imino]-essigsäure werden in 360 ml Acetonitril dispergiert. 14,4 ml (130,5 mMol) N-Methylmorpholin werden unter Rühren hinzugefügt. Nach 10 Minuten werden 34,6 g (103,5 mMol) 2,2-Dithio-bis-benzthiazol hinzugefügt und die erhaltene Suspension auf 0° abgekühlt. Nach Zugabe von 20,2 mi (117 mMol) Triäthylphosphit (langsame Zugabe innerhalb 2 Stunden) wird die Suspension 12 Stunden bei 0° gerührt. Das Produkt wird abfiltriert, nacheinander mit kaltem Acetonitril, Isopropyläther und Petroleumäther gewaschen und bei Zimmertemperatur unter vermindertem Druck getrocknet. Man erhält 2-(2-Amino-4-thiazolyl)-2-[[(Z)-1-(t-butoxycarbonyl)-1-methyläthoxyl]imino]-essigsäure-2-benzthiazolyl-thioester (33,7 g = 81,5 %) vom Schmelzpunkt 139-140°.

### Beispiel 16

a) 17 g (42,7 mMol) Benzyl-(3S,4S)-eis-1-(2,4-dimethoxybenzyl-4-formyl-2-oxo-3-azetidin-carbamat (Beispiel 13 e) werden in 100 ml Methylenchlorid und 100 ml n-Propanol gelöst. Diese Lösung wird mit 3,5 g Hydroxylamin-hydrochlorid (50,3 mMol), gefolgt durch 4,2 ml Pyridin (52 mMol) versetzt. Das Reaktionsgemisch wird unter Rückflussbedingungen 2 Stunden erhitzt. Anschliessend wird das Methylenchlorid abdestilliert und eine Lösung von 6,3g Selendioxid (57 mMol) in 100 ml n-Propanol tropfenweise zugegeben. Das Reaktionsgemisch wird 2 Stunden unter Rückflussbedingungen erhitzt, das Reaktionsgemisch auf Zimmertemperatur abgekühlt und filtriert und die erhaltene Lösung unter vermindertem Druck eingedampft. Das erhaltene Oel wird in 100 ml n-Propanol gelöst und eingedampft. Dieser Vorgang wird zweimal wiederholt. Der erhaltene, teilweise kristalline Rückstand wird in 250 ml Methylenchlorid aufgenommen und nacheinander zweimal mit je 200 ml Wasser und Kochsalzlösung gewaschen. Nach dem Trocknen über Natriumsulfat, Filtration und Abdampfen des Lösungsmittels wird der Rückstand in 70 ml n-Propanol aufgenommen. Die Lösung wird 12 Stunden im Kühlschrank stehengelassen. Man erhält 16,4 g (97 %) Benzyl-(3S,4S)-cis-1-(2,4-dimethoxybenzyl)-4-cyan-2-oxo-3-aze- tidin-carbamat vom Schmelzpunkt 152-153°. [α]_{D} = + 10,6° (c = 1 in Chloroform).
   MS : 395 (M⁺).
b) 15,72 g Kaliumperoxodisulfat (58,2 mMol) und 9,5 g Dikaliumhydrogenphosphat (54,8 mMol) werden in 480 ml Wasser gelöst. Die Lösung wird auf 80° erwärmt und mit einer Lösung von 1,2 g Kupfersulfat in 10 ml Wasser behandelt. Die erhaltene Suspension wird mit 180 ml Acetonitril verdünnt und tropfenweise mit einer Lösung von 14,4 g Benzyl-(3S,4S)-cis-1-(2,4-dimethoxybenzyl)-4-cyan-2-oxo-3-azetidincarbamat in 300 ml Acetonitril behandelt. Das Reaktionsgemisch wird 2 1/2 Stunden unter Rückflussbedingungen erhitzt, anschliessend abgekühlt, filtriert und teilweise eingedampft. Die erhaltene ölige, wässrige Lösung wird mit Aethylacetat extrahiert und die organische Phase nacheinander dreimal mit wässriger gesättigter Natriumbicarbonatlösung, Wasser und Kochsalzlösung gewaschen. Nach dem Trocknen und Abdampfen des Lösungsmittels wird das erhaltene Oel an Kieselgel chromatographiert (230-400 mesh, Eluierungsmittel Aethylacetat : n-Hexan 1 : 1). Man erhält 6,1 g (68,3 %) Benzyl-(3S,4S)-cis-4-cyan-2-oxo-3-azetidincarbamat vom Schmelzpunkt 163-165°.
   MS : 245 (M¹).
c) 6,16 g (25 mMol) Benzyl-(3S,4S)-cis-4-cyan-2-oxo-3-azetidin-carbamat werden in 45 ml Dimethylsulfoxid gelöst und mit 5,58 ml 30 %iger wässriger Wasserstoffperoxid behandelt. Nach Rückgang der Temperatur auf 25° wird das Gemisch mit 5 ml wässriger 1N Natriumhydroxidlösung behandelt. Die Temperatur steigt auf 55°. Nach 45-minütigem Rühren entsteht ein Niederschlag. Es werden 20 ml Aethyiacetat hinzugefügt und die erhaltenen Kristalle abfiltriert. Die Kristalle werden mit wässrigem Aethanol und abs. Aether gewaschen. Man erhält 2,48 g (37,5 %) Benzyl-(3S,4S)-4-carbamoyl-2-oxo-3- azetidin-carbamat vom Schmelzpunkt 248-249 °C. [α]_{D} = + 13° (c = 1 in Dimethylsulfoxid).

Die Mutterlauge wird teilweise eingedampft, wobei weitere 0,53 g Produkt isoliert werden. Die so erhaltene Mutterlauge wird mit Wasser verdünnt und an MCI-Gel (Aethanol-Wasser 3 : 7 als Eluierungsmittel) chromatographiert. Die totale Ausbeute an Endprodukt beträgt 3,5 g (53 %).

d) 7,9 g (30 mMol) Benzyl-(3S,4S)-4-carbamoyl-2-oxo-3-azetidin-carbamat werden in 470 ml abs. Dioxan aufgeschlämmt und mit 6,2 g (39 mMol) Pyridin-Schwefeltrioxid-Komplex behandelt. Die erhaltene Suspension wird 2 Stunden bei Zimmertemperatur gerührt, anschliessend mit 1,41 g (8,8 mMol) Pyridin-Schwefeltrioxid-Komplex behandelt und eine weitere Stunde gerührt. Nach Zugabe von 1,90 g (12 mMol) Pyridin-Schwefeltrioxid-Komplex und weiterem 2-stündigen Rühren wird das Lösungsmittel unter vermindertem Druck eingedampft und der Rückstand in 200 ml Wasser aufgenommen. Die erhaltene wässrige Lösung wird mit 15 g (44,24. mMol) Tetrabutylammoniumhydrogensulfat behandelt. Diese wässrige Lösung wird zweimal mit je 250 ml Methylenchlorid extrahiert, gefolgt durch Trocknen über Natriumsulfat. Nach dem Abdampfen des Lösungsmittels wird der erhaltene ölige Rückstand in 150 ml abs. Methanol gelöst und über 2,5 g 10 % Palladiumkohle hydriert. Der Katalysator wird abfiltriert und die Lösung eingedampft und wieder in eine Lösung von 70 ml Ameisensäure in 100 ml Methylenchlorid gelöst. Nach 2 Stunden wird das Lösungsmittel abgedampft und der Rückstand mit 25 ml Wasser behandelt. Man erhält 2,3 g (36 %) (3S,4S)-3-Amino-4-carbamoyl-2-oxo-1-azetidin-sulfonsäure. Die Mutterlauge wird an MCI-Gel chromatographiert, (Eluierungsmittel: Wasser-Aethanol 1 : bis 9 : 1), wobei weitere 420 mg Produkt erhalten werden. Totalausbeute 2,7 g (43,3 %).
IR (KBr) cm⁻¹ : 1 779, 1 696, 1 633, 1 485, 1 288, 1 250
NMR (d₆DMSO)8(ppm) : 4,43 und 4,72 (2 x 1H,2d,6Hz,CH―CH), 7,88 (2H,d,breit,NH₂), 8,59 (3H,breit,NH₃⁺).

e) Aus (3S,4S)-3-Amino-4-carbamoyl-Z-oxo-1-azetidinsulfonsäure und 2-(2-Amino-4-thiazolyl)-2-(Z)-methoxyimino-essigsäure-2-benzthiazolyl-thioester erhält man in Analogie zu Beispiel 13 I (3S,4S)-3-[(Z)-2-(2-Amino-4-thiazolyl)-2-(methoxyimino)-acetamido]-4-carbamoyl-2-oxo-1-azetidin-sulfonsäure-Natriumsalz.
Elementaranalyse : berechnet für C₁₀H₁₁N₆O₇S₂Na 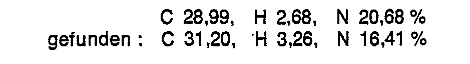
IR (KBr) cm-¹ : 3 282, 1 790, 1 640, 1 612,1 527, 1 260, 1 230
NMR (d₆DMSO)8(ppm): 3,85 (3H,s,OCH₃), 4,3 (1H,d,6Hz,CH―CONH₂), 5,30 (1H,dd,6 und 9Hz, NH―CH), 6,95 (1H,s,S―CH=), 7,40 (2H,d,18Hz,CONH₂), 9,25 (1H,d,9Hz,NH―CO)

Der als Ausgangsverbindung in e) verwendete 2-(2-Amino-4-thiazolyl)-2-(Z)-methoxyimino-essigsäure-2-benzthiazolyl-thioester kann wie folgt hergestellt werden :
f) 3,93 g Triphenylphosphin und 5 g Dithio-bis-benzthiazol werden in 50 ml Dichlormethan suspendiert und etwa 30 Minuten bei Raumtemperatur weitergerührt. Nach Abkühlen auf 0° werden 2 g 2-(2-Amino-4-thiazolyl)-2-(Z)-methoxyimino-essigsäure zugesetzt und 3 bis 4 Stunden bei 0° weitergerührt. Zur Aufarbeitung wird vom Unlöslichen abgenutscht und mit wenig kaltem Methylenchlorid gewaschen. Der Feststoff wird in 25 ml Aethylacetat suspendiert, 30 Minuten bei 0° gerührt, wieder abgesaugt und mit Aethylacetat gewaschen. Man erhält nach dem Umkristallisieren aus Tetrahydrofuran/Dichlormethan 2-(2-Amino-4-thiazolyl)-2-(Z)-methoxyimino-essigsäure-2-benzthiazolyl-thioester vom Schmelzpunkt 128-130°.

### Beispiel 17

a) 265 mg (0,5 mMol) (Z)-2-(2-Amino-4-thiazolyl)2-[[(p-nitrobenzyloxycarbonyl)-methoxy]-imino]-essigsäure-2-benzthiazolyl-thioester und 104 mg (0,5 mMol) (3S,4S)-3-Amino-4-carbamoyl-2-oxo-1-aze- tindisulfonsäure (aus Beispiel 14 d) werden in 2,5 ml abs. Aceton suspendiert und mit 0,15 ml (1,1 mMol) Triäthylamin versetzt. Nach 30 Minuten löst sich die Suspension zu einer gelben Lösung. Nach 24 Stunden bei Zimmertemperatur dampft man ein und chromatographiert den Rückstand mit DCCC (Droplet Counter Current Chromatographie: aufsteigende Tröpfchen im Gemisch CHCl₃ : Me-OH : H₂O = 7 : 13 : 8). Die interessanten Fraktionen werden eingedampft und lyophilisiert. Man erhält 0,139 g (41 %) (3S,4S)-3-[(Z)-2-(2-amino-4-thiazolyl)-2-[[(p-nitrobenzyloxycarbonyl)-methoxyimino]aceta- mido]-4-carbamoyl-2-oxo-1-azetidinsulfonsäure-triäthytaminsalz.
   NMR (D₂O)δ(ppm) : 1,31 (t, J = 7,5, 9H), 3,24 (q, J = 7,5, 6H), 4,95 (d, J = 6,0 1 H), 4,95 (S,2H), 5,34 (S,2H), 5,69 (d, J ₌ 6,0, 1 H), 6,98 (s,1 H), 7,49 (d, J = 9,0, 2H), 8,16 (d, J = 9,0, 2H)
   IR (KBr) cm⁻¹ : 3333 (42 %), 1 773 (26 %), 1 687 (20 %), 1 608 (42 %), 1 348 (27 %), 1 277 (22 %), 1 248 (26 %), 1 046 (19 %) 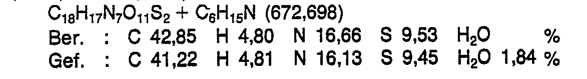
b) 336 mg (0,5 mMol) (3S,4S)-3-[(Z)-2-(2-amino-4-thiazolyl)-2-[[(p-nitrobenzyloxycarbonyl)-methoxyl- mino]acetamido]-4-carbamoyl-2-oxo-1-azetidinsulfonsäure-triäthylaminsalz werden in 20 ml Methanol gelöst und mit 150 mg 5 % Palladium auf Kieselgur 3-4 Stunden bei Zimmertemperatur hydriert. Der Katalysator wird durch Filtration entfernt und die Lösung eingedampft. Der Rückstand wird in wenig gesättigter wässriger Natriumbicarbonatlösung gelöst und an Amberlite XAD-2 chromatographiert (Eluens : Wasser, anschliessend 40 % Aethanol in Wasser). Nach, Lyophilisierung werden 150 mg (65 %) (3S,4S)-3-[(Z)-2-(2-Amino-4-thiazolyl)-2-[(carboxymethoxy)imino]acetamido]-4-carbamoyl-2-oxo-1-azeti- dinsulfonsäure-Natriumsalz erhalten.
   IR : 3 421, 1 769, 1 731, 1 690 cm⁻¹

Der als Ausgangsverbindung in a) verwendete 2-(2-Amino-4-thiazolyl)-2-[[(Z)-(p-nitrobenzyloxycarbonyl)-methoxy]-imino]-essigsäure-2-benzthiazolyl-thioester kann wie folgt hergestellt werden .

c) 6,1 g (25 mMol) 2-(2-Amino-4-thiazolyl)-2-(Z)-hydroxyimino-essigsäure-t-butylester werden in 250 ml trockenem Acetonitril dispergiert. Es werden nun 13,7 g (50 mMol) Bromessigsäure-4-nitrobenzy- lester und 12,9 ml (75 mMol) N-Aethyldiisopropylamin unter Rühren bei Zimmertemperatur hinzugefügt. 5 Minuten später werden 7,5 g (50 mMol) Natriumjodid hinzugefügt. Das Reaktionsgemisch wird 3 1/2 Stunden in einer Argonatmosphäre bei Zimmertemperatur gerührt. Anschliessend wird das Lösungsmittel abgedampft und der Rückstand mit 500 ml Aethylacetat verdünnt. Die erhaltene Lösung wird viermal mit insgesamt 2 I Wasser gewaschen. Das Wasser wird mit 300 ml Aethylacetat extrahiert und die vereinigten Aethylacetatlösungen über Natriumsulfat getrocknet und zur Trockene eingedampft. Nach dem Kristallisieren aus Aethylacetat-Hexan erhält man 8,2 g (75 %) 2-(2-Amino-4-thiazolyl)-2-[[(p-nitrobenzyloxycarbonyl)-methoxy]-imino]-essigsäure-t-butylester vom Schmelzpunkt 146,8 °C (Zers.).
d) 5,0 g (11,4 mMol) 2-(2-Amino-4-thiazolyl)-2-[[(Z)-(p-nitrobenzyloxycarbonyl)-methoxy]-imino]-essigsäure-t-butylester werden in 86 ml Essigsäure gerührt und mit 5,2 ml (38,4 mMol) Bortrifluoridätherat versetzt. Die erhaltene Lösung wird 5 Stunden bei Zimmertemperatur gerührt und anschliessend auf 260 ml Wasser gegossen. Der erhaltene Niederschlag wird abfiltriert und bei 40 °C unter vermindertem Druck getrocknet. Man erhält 3,5 g (80 %) 2-(2-Amino-4-thiazolyl)-2-[[(p-nitrobenzyloxycarbonyl)-metho- xy]-imino]-essigsäure. Schmelzpunkt etwa 175 °C (Zers.).
e) 1,9 g (0,5 mMol) 2-(2-Amino-4-thiazolyl)-2-[[(Z)-(p-nitrobenzyloxycarbonyl)-methoxy]-imino]-essigsäure werden in 30 ml Acetonitril (getrocknet mit einem 3 Ä Molekularsieb) dispergiert. Diese Suspension wird mit 1,4 ml (12,7 mMol) N-Methylmorpholin unter Rühren versetzt, gefolgt durch 2,0 g (6,0 mMol) 2,2-Dithio-bis-benzthiazol und 1,14 ml (6,7 mMol) Triäthylphosphit. Nach 1-stündigem Rühren bei Zimmertemperatur wird das Reaktionsgemisch auf 0 °C abgekühlt und filtriert. Das Filtrat wird eingedampft und der Rückstand aus Methylenchlorid kristallisiert. Man erhält 1,03 g (39 %) 2-(2-Amino-4-thiazolyl)-2-[[(Z)-(p-nitrobenzyloxycarbonyl)-methoxy]-imino]-essigsäure-2-benzthiazolyl-thioester vom Schmelzpunkt 124-126 °C.

### Beispiel 18

a) In Analogie zu Beispiel 15 a erhält man aus 2-(2-Amino-4-thiazolyl)-2-[[(Z)-(p-nitrobenzyloxycarbonyl)-methoxy]-imino]-essigsäure-2-benzthiazolyl-thioester und (3S,4S)-3-Amino-4-carbamoyloxy-methyl-2-oxo-1-azetidinsulfonsäure das (3S,4S)-3-[(Z)-2-(2-Amino-4-thiazolyl)-2-[[(p-nitrobenzyloxycarbonyl)methoxyimino]acetamido]-4-carbamoyloxymethyl-2-oxo-1-azetidin-sulfonsäure-Natriumsalz.
   Berechnet für C₁₉H₁₈N₇O₁₂S₂Na 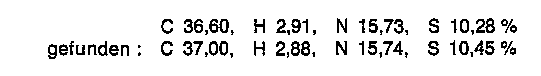
   IR (KBr) cm-¹ : 3 353, 1 761, 1 729, 1 524, 1 348
   NMR (d₆DMSO)δ(ppm) : 4,0-4,2 (3H,m,CH―CH₂), (2H,s,∅―CH₂), 5,30 (1H,dd,NH―CH―), 5,32 (2H,s,O―CH₂), 6,70 (2H,breit,NH₂), 6,9 (1H,s,S―CH=), 7,10 (2H,breit,NH₂), 7,70 und 8,2 (2 x 2H,2d,3Hz,Ar), 9,5 (1 H,d,9Hz,NHCO)
b) 270 mg (0,43 mMol) (3S,4S)-3-[(Z)-2-(2-Amino-4-thiazolyl)-2-[[(p-nitrobenzyloxycarbonyl)methoxy- imino]acetamido]-4-carbamoyloxymethyl-2-oxo-1-azetidin-sulfonsäure-Natriumsalz werden in 30 ml Methanol gelöst und über 5 % Palladium auf Kieselgur (150 mg) hydriert. Der Katalysator wird abfiltriert und das Lösungsmittel abgedampft. Der Rückstand wird in 2,5 ml Wasser aufgenommen und zweimal mit Aethylacetat gewaschen. Die wässrige Phase wird chromatographiert (Reverse Phase, Wasser als Eluierungsmittel). Man erhält 115 mg (54 %) (3S,4S)-3-[(Z)-2-(2-Amino-4-thiazolyl)-2-[(carboxymethoxy)i-mino]acetamido]-4-carbamoyloxymethyl-2-oxo-1-azetidin-sulfonsäure-Natriumsalz.
   Elementaranalyse : berechnet für C₁₂H₁₃N₆O₁₀S₂NA 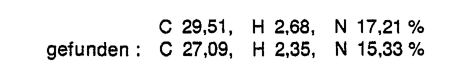
   IR (KBr) cm-¹ : 3 434, 1 766, 1 718, 1 669, 1 613, 1 533, 1 278, 1 251
   NMR (dₑDMSO)8(ppm): 3,90-4,15 (3H,m,CH―CH₂), 4,30 (2H,s,CH₂―COOH), 5,20 (lH,dd,5 und 9Hz,NH―CH), 6,6 (2H,breit,NH₂), 6.78 (1 H,s,S―CH=), 7,13 (2H,s,NH₂), 10,90 (1 H,d,9Hz,CONH)

### Beispiel 19

In Analogie zu den Beispielen 15 und 16 erhält man ebenfalls (3S,4S)-3-[(Z)-2-[2-Amino-4-thiazolyl)-2-[1-carboxy-1-methyläthoxy]imino]acetamido]-4-carbamoyl-2-oxo-1-azetidin-sulfonsäure.
Elementaranalyse : berechnet für C₁₃H₁₆N₆O₉S₂ 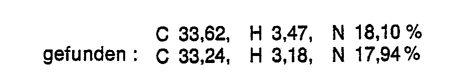
IR (KBr) cm⁻¹ : 3 332, 3 208, 2 552, 1 780, 1 684, 1 638, 1 279, 1 188
NMR (d₆DSMO)δ(ppm) : 1,44 (6H,s,2 × CH₃), 4,34 (1H,d,6Hz,CH―CONH₂), 5,33 (lH,dd,6 und 9Hz,NH―CH―), 6,96 (1H,s,S―CH=), 7,40 (2H,breit,d,7Hz,CONH₂), 8,95 (1H,d,9Hz,CONH)
UV (EtOH) : 292 nm (6 846), 240 nm (12 232).

## Patentansprüche (Patentansprüche für folgende(n) Vertragsstaat(en) : BE, CH, DE, FR, GB, IT, LI, LU, NL, SE)

1. Optisch einheitliche ß-Lactame der allgemeinen Formel 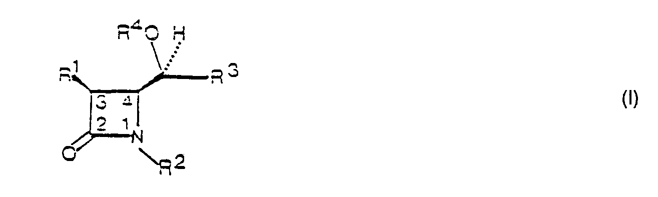 worin R¹ Amino, Azido, Phthalimido oder die Gruppe ROCO-CH=C(CH₃)-NH-, oder Z-NH-, R niederes Alkyl, Z gegebenenfalls durch Chlor substituiertes niederes Akoxycarbonyl, Benzyloxycarbonyl oder Benzhydryl, R² Wasserstoff oder die Gruppe Z'-, Z' 2,4- oder 3,4-Di(niederes Alkoxy)benzyl, 2,4-oder 3,4-Di(niederes Alkoxy)phenyl, Di[4-(niederes Alkoxy)-phenyl]methyl oder 4-(niederes Alkoxy)phenyl, R³ niederes Alkyl, Phenyl-niederes Alkyl, niederes Alkoxy-niederes Alkyl, insbesondere niederes Alkoxymethyl, und R⁴ niederes Alkyl oder Phenyl-niederes Alkyl oder R³ und R⁴ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden 0-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, bedeuten, wobei R¹ eine Gruppe der Formel Z-NH- bedeutet, wenn R² Wasserstoff bedeutet, und die mit nieder bezeichneten Reste bis zu 7 Kohlenstoffatome besitzen, und die entsprechenden optischen Antipoden davon.

2. Verbindungen gemäss Anspruch 1, worin R³ und R⁴ zusammen mit dem Chiralitätszentrum eine Gruppe der Formel 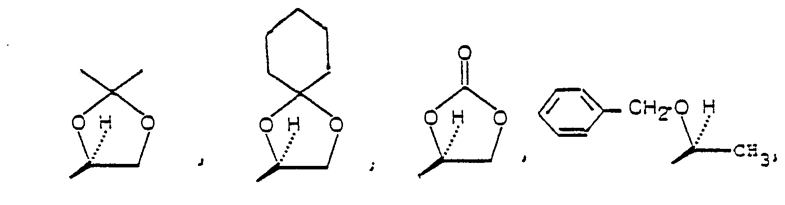 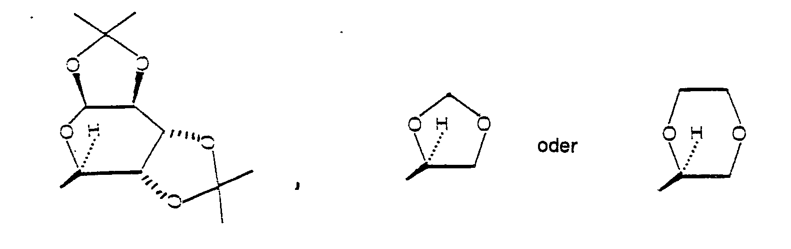 bedeuten.

3. N-[(2R,3R)-1-(2,4-dimethoxybenzyl)-4-[(S)-1,4-dioxaspiro-[4.5]dec-2-yl]-2-oxo-3-azetidinyl]phthali- mid.

4. N-[(3S,4S)-cis-1-(3,4-Dimethoxybenzyl)-4-[(R)-2,2-dimethyl-1,3-dioxolan-4-yl]-2-oxo-3-azetidi- nyl]phthalimid.

5. N-[(3S,4S)-4-[(S)-1-(Benzyloxy)äthyl]-1-(2,4-dimethoxybenzyl)-2-oxo-3-azetidinyl]phthalimid.

6. Verbindungen gemäss Anspruch 1, worin R³ und R⁴ zusammen mit dem Chiralitätszentrum die Gruppe 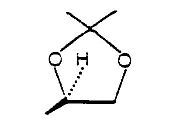 bedeuten, und die entsprechenden optischen Antipoden davon.

7. Verbindungen gemäss Anspruch 6, worin R¹ Phthalimido, Amino oder Benzyloxycarbonylamino und R² Wasserstoff oder 2,4-Dimethoxybenzyl bedeuten.

8. N-[(3S,4S)-cis-1-(2,4-Dimethoxybenzyl)-4-[(R)-2,2-dimethyl-1,3-dioxolan-4-yl]-2-oxo-3-azetidi- nyl]phthalimid.

9. (3S,4S)-cis-3-Amino-1-(2,4-dimethoxybenzyl)-4-[(R)-2,2-dimethyl-1,3-dioxolan-4-yi]-2-azetidinon.

10. Benzyl-(3S,4S)-cis-1-(2,4-dimethoxybenzyl)-4-[(R)-2,2-dimethyl-1,3-dioxolan-4-yl]-2-oxo-3-azeti- dincarbamat.

11. Benzyl-(3S,4S)-cis-4-[(R)-2,2-dimethyl-1,3-dioxolan-4-yl]-2-oxo-3-azetidincarbamat.

12. Optisch einheitliche β-Lactame der allgemeinen Formel 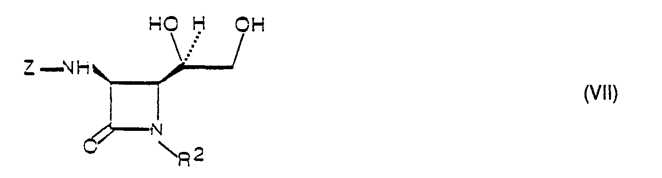 worin Z und R² die in Anspruch 1 angegebene Bedeutung besitzen, und die entsprechenden optischen Antipoden davon.

13. Verbindungen gemäss Anspruch 12, worin Z Benzyloxycarbonyl und R² Wasserstoff oder 2,4-Dimethoxybenzyl bedeuten.

14. Benzyl-(3S,4S)-cis-4-[(R)-1,2-dihydroxyäthyl]-1-(2,4-dimethoxybenzyl)-2-oxo-3-azetidincarbamat.

15. Optisch einheitliche β-Lactame der allgemeinen Formel 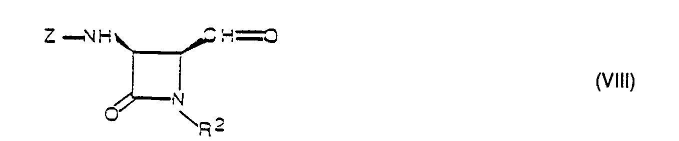 worin Z und R² die in Anspruch 1 angegebene Bedeutung besitzen, und die entsprechenden optischen Antipoden davon.

16. Verbindungen gemäss Anspruch 15, worin Z Benzyloxycarbonyl und R² Wasserstoff oder 2,4-Dimethoxybenzyl bedeuten.

17. Benzyl-(3S,4S)-cis-1-(2,4-dimethoxybenzyl)-4-formyl-2-oxo-3-azetidincarbamat.

18. Benzyl-(3S;4S)-cis-4-formyl-2-oxo-3-azetidincarbamat.

19. Verfahren zur Herstellung von Verbindungen gemäss einem der Ansprüche 1-11, dadurch gekennzeichnet, dass man ein reaktives Derivat einer Carbonsäure der allgemeinen Formel
R¹¹―CH₂―COOH (II)
worin R¹¹ Azido, Phthalimido oder die Gruppe ROCO―CH=C(CH₃)―NH― und R niederes Alkyl bedeuten, in Gegenwart einer Base mit einer optisch einheitlichen Verbindung der allgemeinen Formel 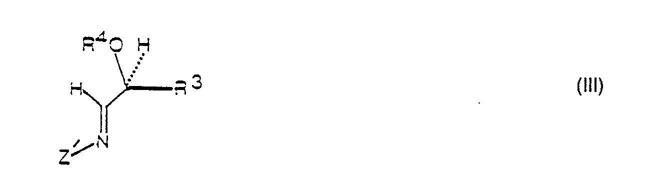 worin R³, R⁴ und Z' die in Anspruch 1 angegebene Bedeutung besitzen, oder dem optischen Antipoden davon umsetzt, erwünschtenfalls in einer so erhaltenen Verbindung der allgemeinen Formel 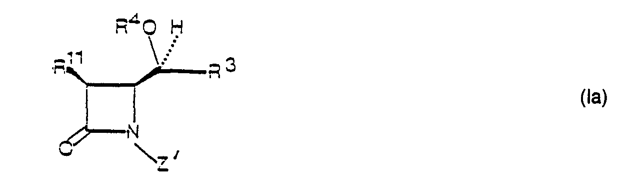 worin R", R³, R⁴ und Z' obige Bedeutung besitzen, oder dem optischen Antipoden davon die Gruppe R¹¹ in die Aminogruppe umwandelt, erwünschtenfalls eine so erhaltene Verbindung der allgemeinen Formel 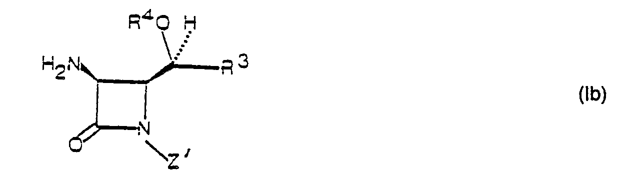 worin R³, R⁴ und Z' obige Bedeutung besitzen, oder den optischen Antipoden davon mit einem die Gruppe Z liefernden Mittel umsetzt und erwünschtenfalls in einer so erhaltenen Verbindung der allgemeinen Formel 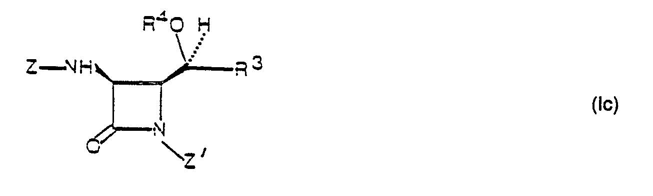 worin R³, R⁴ und Z' obige und Z die in Anspruch 1 angegebene Bedeutung besitzen, oder dem optischen Antipoden davon die mit Z' bezeichnete Gruppe abspaltet.

20. Verfahren zur Herstellung von optisch einheitlichen β-Lactamen der allgemeinen Formel 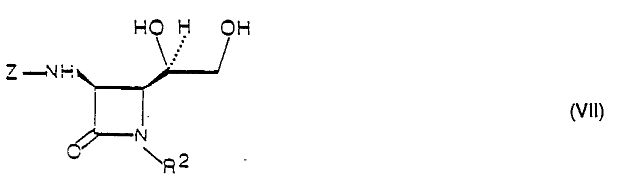 worin Z und R² die in Anspruch 1 angegebene Bedeutung besitzen, und von entsprechenden optischen Antipoden davon, dadurch gekennzeichnet, dass man in einer gemäss Anspruch 19 erhaltenen Verbindung der allgemeinen Formel 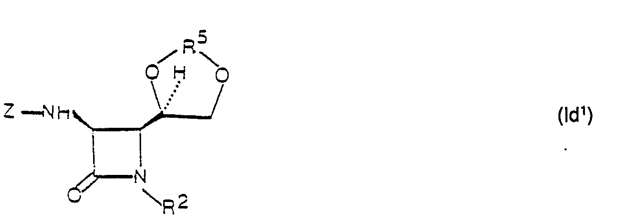 worin R⁵ Methylen, Aethylen, Oxomethylen, Cyclohexyliden oder Isopropyliden bedeutet, und Z und R² obige Bedeutung besitzen, bzw. dem optischen Antipoden davon den Rest R⁵ abspaltet.

21. Verfahren zur Herstellung von optisch einheitlichen β-Lactamen der allgemeinen Formel 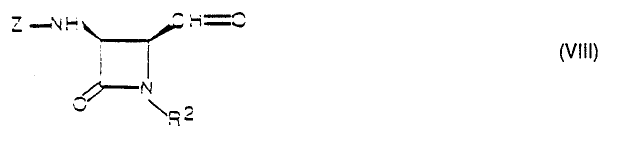 worin Z und R² die in Anspruch 1 angegebene Bedeutung besitzen, bzw. von entsprechenden optischen Antipoden davon, dadurch gekennzeichnet, dass man in einer gemäss Anspruch 20 erhaltenen Verbindung die Diolgruppierung spaltet.

22. Verfahren zur Herstellung von optisch einheitlichen β-Lactamen der allgemeinen Formel 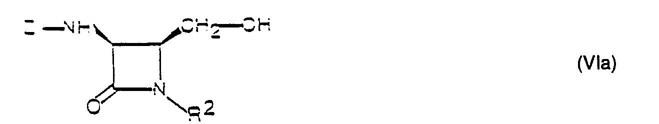 worin Z und R² die in Anspruch 1 angegebene Bedeutung besitzen, bzw. von entsprechenden optischen Antipoden davon, dadurch gekennzeichnet, dass man eine gemäss Anspruch 21 erhaltene Verbindung reduziert.

## Patentansprüche (Patentansprüche für folgende(n) Vertragsstaat(en) : AT)

1. Verfahren zur Herstellung von optisch einheitlichen β-Lactamen der allgemeinen Formel 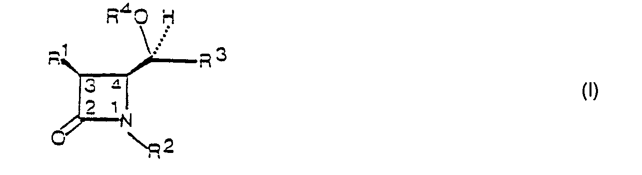 worin R¹ Amino, Azido, Phthalimido oder die Gruppe ROCO―CH=C(CH₃)―NH― oder Z―NH―, R niederes Alkyl, Z gegebenenfalls durch Chlor substituiertes niederes Alkoxycarbonyl, Benzyloxycarbonyl oder Benzhydryl, R² Wasserstoff oder die Gruppe Z-, Z 2,4- oder 3,4-Di(niederes Alkoxy)benzyl, 2,4-oder 3,4-Di(niederes Alkoxy)phenyl, Di[4-(niederes Alkoxy)-phenyl]methyl oder 4-(niederes Alkoxy)phenyl, R³ niederes Alkyl, Phenyl-niederes Alkyl, niederes Alkoxy-niederes Alkyl, insbesondere niederes Alkoxymethyl, und R⁴ niederes Alkyl oder Phenyl-niederes Alkyl oder R³ und R⁴ zusammen mit dem Chiralitätszentrum einen 5- oder 6-gliedrigen, gegebenenfalls ein weiteres, mit dem Chiralitätszentrum nicht direkt verbundenes Sauerstoffatom enthaltenden O-Heterocyclus, der gegebenenfalls durch niederes Alkyl, niederes Alkoxy, Oxo oder Spirocyclo-niederes Alkyl substituiert sein kann, wobei benachbarte niedere Alkoxygruppen zusammen einen Ring bilden können, wie z. B. bei Isopropylidendioxy, bedeuten, wobei R' eine Gruppe der Formel Z-NH- bedeutet, wenn R² Wasserstoff bedeutet, und die mit nieder bezeichneten Reste bis zy 7 Kohlenstoffatome besitzen, und von entsprechenden optischen Antipoden davon, dadurch gekennzeichnet, dass man ein reaktives Derivat einer Carbonsäure der allgemeinen Formel 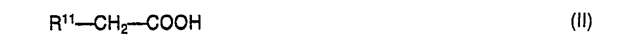 worin R¹¹ Azido, Phthalimido oder die Gruppe ROCO―CH=C(CH₃)―NH― und R niederes Alkyl bedeuten, in Gegenwart einer Base mit einer optisch einheitlichen Verbindung der allgemeinen Formel 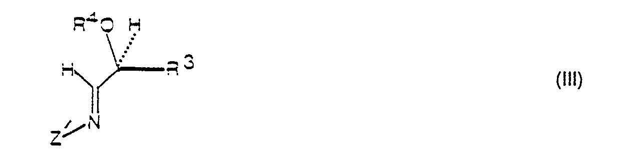 worin R³, R⁴ und Z' obige Bedeutung besitzen, oder dem optischen Antipoden davon umsetzt, erwünschtenfalls in einer so erhaltenen Verbindung der allgemeinen Formel 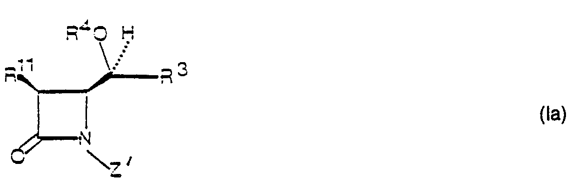 worin R", R³, R⁴ und Z' obige Bedeutung besitzen, oder dem optischen Antipoden davon die Gruppe R" in die Aminogruppe umwandelt, erwünschtenfalls eine so erhaltene Verbindung der allgemeinen Formel 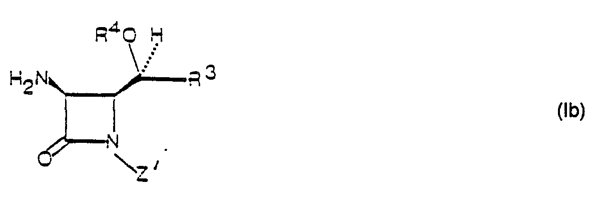 worin R³, R⁴ und Z' obige Bedeutung besitzen, oder den optischen Antipoden davon mit einem die Gruppe Z liefernden Mittel umsetzt und erwünschtenfalls in einer so erhaltenen Verbindung der allgemeinen Formel 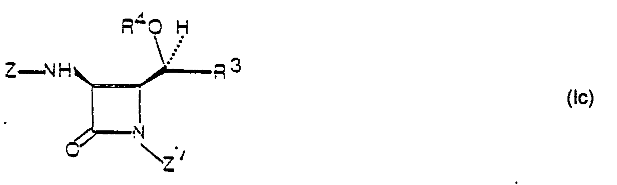 worin R³, R⁴, Z und Z' obige Bedeutung besitzen, oder dem optischen Antipoden davon die mit Z' bezeichnete Gruppe abspaltet.

2. Verfahren nach Anspruch 1, dadurch gekennzeichnet, dass man als reaktives Derivat der Carbonsäure der Formel II das entsprechende Carbonsäurehalogenid, insbesondere das Chlorid, einsetzt.

3. Verfahren nach Anspruch 1 oder 2, dadurch gekennzeichnet, dass man eine Ausgangsverbindung der Formel III bzw. den optischen Antipoden davon einsetzt, worin R³ und R⁴ zusammen mit dem Chiralitätszentrum eine Gruppe der Formel 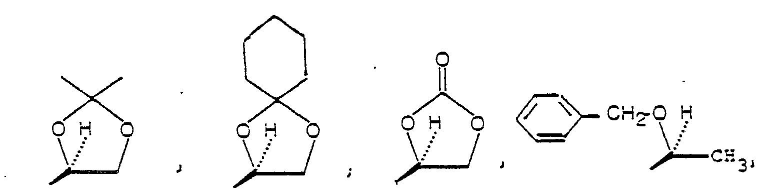 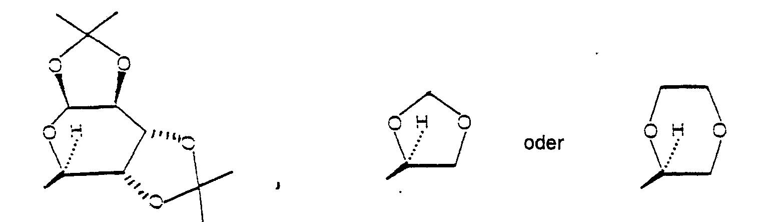 bedeuten.

4. Verfahren nach einem der Ansprüche 1-3, dadurch gekennzeichnet, dass man N-[(2R, 3R)-1-(2,4-dimethoxybenzyl)-4-[(S)-1,4-dioxaspiro[4.5]dec-2-yl]-2-oxo-3-azetidinyl]-phthalimid herstellt.

5. Verfahren nach einem der Ansprüche 1-3, dadurch gekennzeichnet, dass man N-[(3S, 4S)-cis-1-(3,4-Dimethoxybenzyl)-4-[(R)-2,2-dimethyl-1,3-dioxolan-4-yl]-2-oxo-3-azetidinyl]phthalimid herstellt.

6. Verfahren nach einem der Ansprüche 1-3, dadurch gekennzeichnet, dass man N-[(3S, 4S)-4-[(S)-1-(Benzyloxy)-äthyl]-1-(2,4-dimethoxybenzyl)-2-oxo-3-azetidinyl]phthalimid herstellt.

7. Verfahren nach Anspruch 1, dadurch gekennzeichnet, dass R³ und R⁴ zusammen mit dem Chiralitätszentrum die Gruppe 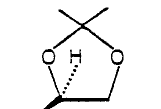 bedeuten.

8. Verfahren nach Anspruch 7, dadurch gekennzeichnet, dass R¹ Phthalimido, Amino oder Benzyloxycarbonylamino und R² 2,4-Dimethoxybenzyl bedeuten.

9. Verfahren nach Anspruch 7 oder 8, dadurch gekennzeichnet, dass man N-[(3S, 4S)-cis-1-(2,4-Dimethoxybenzyl)-4-[(R)-2,2-dimethyl-1,3-dioxolan-4-yl]-2-oxo-3-azetidinyl]phthalimid, (3S, 4S)-cis-3-Amino-1-(2,4-dimethoxybenzyl)-4-[(R)-2,2-dimethyl-1,3-dioxolan-4-yl]-2-azetidinon, Benzyl-(3S, 4S)-cis-1-(2,4-dimethoxybenzyl)-4-[(R)-2,2-dimethyl-1,3-dioxolan-4-yl]-2-oxo-3-azetidincarbamat und/oder Benzyl-(3S, 4S)-cis-4-[(R)-2,2-dimethyl-1,3-dioxolan-4-yl]-2-oxo-3-azetidincarbamat herstellt.

10. Verfahren zur Herstellung von optisch einheitlichen β-Lactamen der allgemeinen Formel 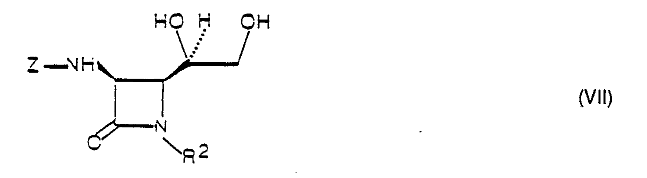 worin Z und R² die in Anspruch 1 angegebene Bedeutung besitzen, und von entsprechenden optischen Antipoden davon, dadurch gekennzeichnet, dass man in einer gemäss Anspruch 1 erhaltenen Verbindung der allgemeinen Formel 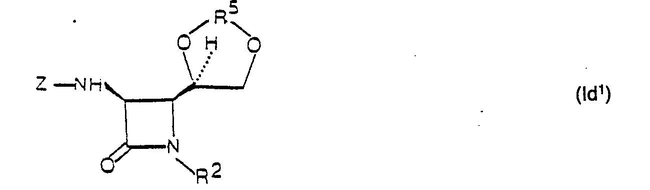 worin R⁵ Methylen, Aethylen, Oxomethylen, Cyclohexyliden oder Isopropyliden bedeutet, und Z und R² obige Bedeutung besitzen, bzw. dem optischen Antipoden davon den Rest R⁵ abspaltet.

11. Verfahren nach Anspruch 10, dadurch gekennzeichnet, dass man die erhaltene Verbindung der Formel Id¹ bzw. den optischen Antipoden davon mit einem mild sauren Agens, z. B. mit einem sulfonierten Ionenaustauscher, Pyridinium-p-toluolsulfonat oder p-Toluolsulfonsäure, behandelt.

12. Verfahren gemäss Anspruch 10 oder 11, dadurch gekennzeichnet, dass R⁵ Isopropyliden bedeutet.

13. Verfahren gemäss einem der Ansprüche 10 bis 12, dadurch gekennzeichnet, dass Z Benzyloxycarbonyl und R² Wasserstoff oder 2,4-Dimethoxybenzyl bedeuten.

14. Verfahren gemäss einem der Ansprüche 10 bis 13, dadurch gekennzeichnet, dass man Benzyl-(3S, 4S)-cis-4-[(R)-1,2-dihydroxyäthyl]-1-(2,4-dimethoxybenzyl)-2-oxo-3-azetidincarbamat herstellt.

15. Verfahren zur Herstellung von optisch einheitlichen β-Lactamen der allgemeinen Formel 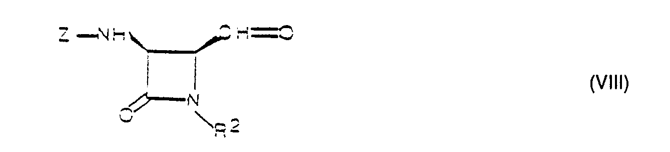 worin Z und R² die in Anspruch 1 angegebene Bedeutung besitzen, bzw. von entsprechenden optischen. Antipoden davon, dadurch gekennzeichnet, dass man in einer gemäss Anspruch 10 erhaltenen Verbindung die Diolgruppierung spaltet.

16. Verfahren nach Anspruch 15, dadurch gekennzeichnet, dass man die Spaltung mit Hilfe von Natriummetaperjodat durchführt.

17. Verfahren nach Anspruch 15 oder 16, dadurch gekennzeichnet, dass man Benzyl-(3S, 4S)-cis-1-(2,4-dimethoxybenzyl)-4-formyl-2-oxo-3-azetidincarbamat herstellt.

18. Verfahren nach Anspruch 15 oder 16, dadurch gekennzeichnet, dass man Benzyl-(3S, 4S)-cis-4- formyl-2-oxo-3-azetidincarbamat herstellt.

19. Verfahren zur Herstellung von optisch einheitlichen β-Lactamen der allgemeinen Formel 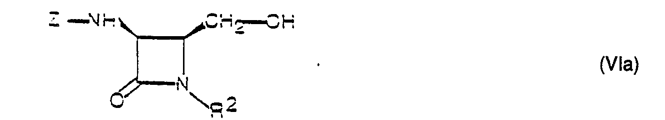 worin Z und R² die in Anspruch 1 angegebene Bedeutung besitzen, bzw. von entsprechenden optischen Antipoden davon, dadurch gekennzeichnet, dass man eine gemäss Anspruch 15 erhaltene Verbindung reduziert.

20. Verfahren nach Anspruch 19, dadurch gekennzeichnet, dass man die Reduktion mit Natriumborhydrid in einem niederen Alkanol durchführt.

21. Verfahren nach Anspruch 19 oder 20, dadurch gekennzeichnet, dass man Benzyl-(3S, 4S)-cis-1-(2,4-dimethoxybenzyl)-4-hydroxymethyl-2-oxo-3-azetidincarbamat herstellt.

## Claims (Claims for the following Contracting State(s) : BE, CH, DE, FR, GB, IT, LI, LU, NL, SE)

1. Optically uniform β-lactams of the general formula 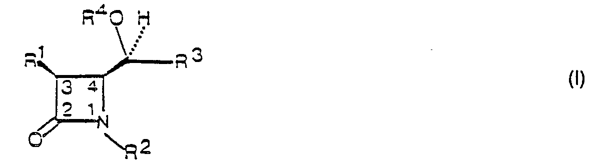 wherein R¹ signifies amino, azido, phthalimido or the group ROCO-CH=C(CH₃)-NH- or Z-NH-, R signifies lower alkyl, Z signifies lower alkoxycarbonyl optionally substituted by chlorine, benzyloxycarbonyl or benzhydryl, R² signifies hydrogen or the group Z'-, Z' signifies 2,4- or 3,4-di(lower alkoxy)benzyl, 2,4- or 3,4-di(lower alkoxy)phenyl, di[4-(lower alkoxy)phenyl]methyl or 4-(lower alkoxy)phenyl, R³ signifies lower alkyl, phenyl-lower alkyl, lower alkoxy-lower alkyl, especially lower alkoxymethyl, and R⁴ signifies lower alkyl or phenyl-lower alkyl or R³ and R⁴ together with the centre of chirality signify a 5- or 6-membered O-heterocycle which optionally contains a further oxygen atom not directly linked with the centre of chirality and which can be substituted, if desired, by lower alkyl, lower alkoxy, oxo or spirocyclo-lower alkyl, whereby adjacent lower alkoxy groups can together form a ring, such as e. g. in the case of isopropylidenedioxy, whereby R¹ signifies a group of the formula Z-NHwhen R² signifies hydrogen, and the residues denoted by lower have up to 7 carbon atoms, and the corresponding optical antipodes thereof.

2. Compounds in accordance with claim 1, wherein R³ and R⁴ together with the centre of chirality signify a group of the formula

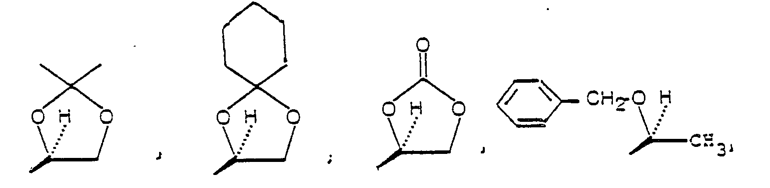

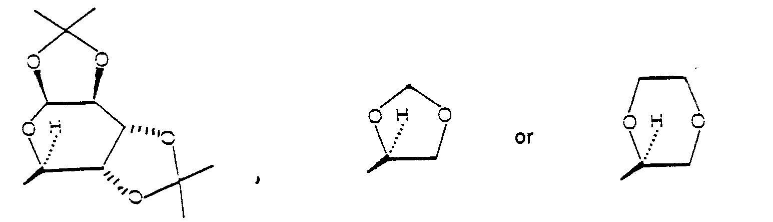

3. N-[(2R,3R)-1-(2,4-Dimethoxybenzyl)-4-[(S)-1,4-dioxaspiro[4.5]dec-2-yl]-2-oxo-3-azetidinyl] phthalimide.

4. N-[(3S,4S)-cis-1-(3,4-Dimethoxybenzyl)-4-[(R)-2,2-dimethyl-1,3-dioxolan-4-yl]-2-oxo-3-azetidinyl] phthalimide.

5. N-[(3S, 4S)-4-[(S)-1-(Benryloxy)ethyl]-1-(2,4-dimethoxybenzyl)-2-oxo-3-azetidinyl]phthalimide.

6. Compounds in accordance with claim 1, wherein R³ and R⁴ together with the centre of chirality signify the group 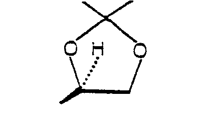 and the corresponding optical antipodes thereof,

7. Compounds in accordance with claim 6, wherein R¹ signifies phthalimido, amino or benzyloxycarbonylamino and R² signifies hydrogen or 2,4-dimethoxybenzyl.

8. N-[(3S,4S)-cis-1-(2,4-Dimethoxybenzyl)-4-[(R)-2,2-dimethyl-1,3-dioxolan-4-yl]-2-oxo-3-azetidinyl] phthalimide.

9. (3S, 4S)-cis-3-Amino-1-(2,4-dimethoxybenzyl)-4-[(R)-2,2-dimethyl-1,3-dioxolan-4-yl]-2-azetidinone.

10. Benzyl (3S, 4S)-cis-1-(2,4-dimethoxybenzyl)-4-[(R)-2,2-dimethyl-1,3-dioxolan-4-yi]-2-oxo-3- azetidinecarbamate.

11. Benzyl (3S, 4S)-cis-4-[(R)-2,2-dimethyl-1,3-dioxolan-4-yl]-2-oxo-3-azetidinecarbamate.

12. Optically uniform β-lactams of the general formula 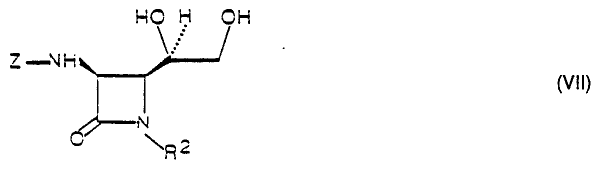 wherein Z and R² have the significance given in claim 1, and the corresponding optical antipodes thereof.

13. Compounds in accordance with claim 12, wherein Z signifies benzyloxycarbonyl and R² signifies hydrogen or 2,4-dimethoxybenzyl.

14. Benzyl (3S, 4S)-cis-4-[(R)-1,2-dihydroxyethyl]-1-(2,4-dimethoxybenzyl)-2-oxo-3-azetidinecar- bamate.

15. Optically uniform β-lactams of the general formula 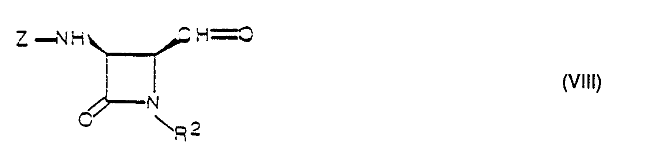 wherein Z and R² have the significance given in claim 1, and the corresponding optical antipodes thereof.

16. Compounds in accordance with claim 15, wherein Z signifies benzyloxycarbonyl and R² signifies hydrogen or 2,4-dimethoxybenzyl.

17. Benzyl (3S, 4S)-cis-1-(2,4-dimethoxybenzyl)-4-formyl-2-oxo-3-azetidinecarbamate.

18. Benzyl (3S, 4S)-cis-4-formyl-2-oxo-3-azetidinecarbamate.

19. A process for the manufacture of compounds in accordance with any one of claims 1-11, characterized by reacting a reactive derivative of a carboxylic acid of the general formula 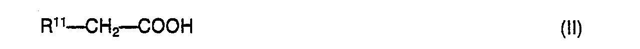 wheren R" signifies azido, phthalimido or the group ROCO-CH=C(CH₃)-NH- and R signifies lower alkyl, in the presence of a base with an optically uniform compound of the general formula 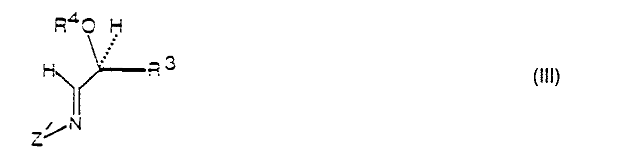 wherein R³, R⁴ and Z' have the significance given in claim 1, or the optical antipode thereof, if desired converting the group R¹¹ in a thus-obtained compound of the general formula 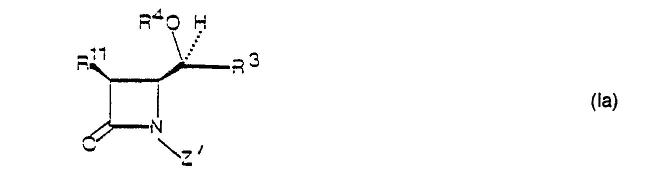 wherein R", R³, R⁴ and Z' have the above significance, or the optical antipode thereof into the amino group, if desired reacting a thus-obtained compound of the general formula 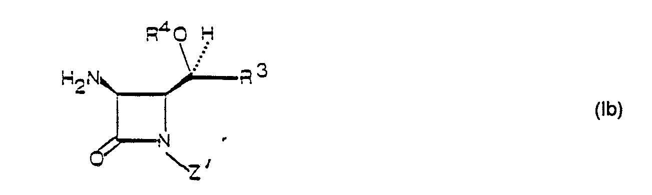 wherein R³, R⁴ and Z' have the above significance, or the optical antipode thereof with an agent yielding the group Z and, if desired, cleaving off the group denoted by Z' in a thus-obtained compound of the general formula 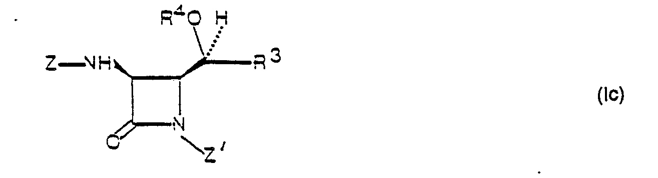 wherein R³, R⁴ and Z' have the significance given above and Z has the significance given in claim 1, orthe optical antipode thereof.

20. A process for the manufacture of optically uniform β-lactams of the general formula 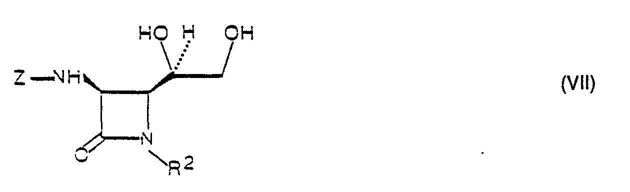 wherein Z and R² have the significance given in claim 1, and of corresponding optical antipodes thereof, characterized by cleaving off the residue R⁵ in a compound obtained in accordance with claim 19 of the general formula 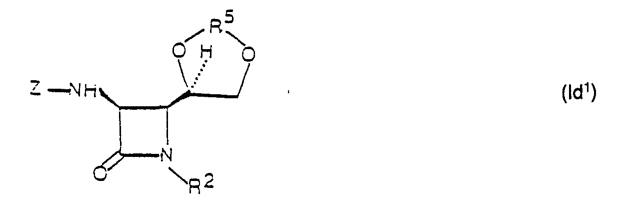 wherein R⁵ signifies methylene, ethylene, oxomethylene, cyclohexylidene or isopropylidene and Z and R² have the above significance, or the optical antipode thereof.

21. A process for the manufacture of optically uniform β-lactams of the general formula 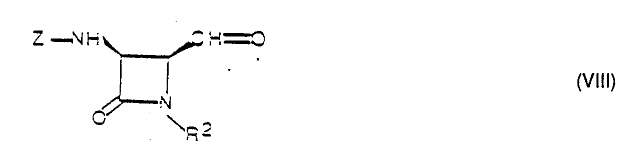 wherein Z and R² have the significance given in claim 1, or of corresponding optical antipodes thereof, characterized by cleaving the diol grouping in a compound obtained in accordance with claim 20.

22. A process for the manufacture of optically uniform β-lactams of the general formula 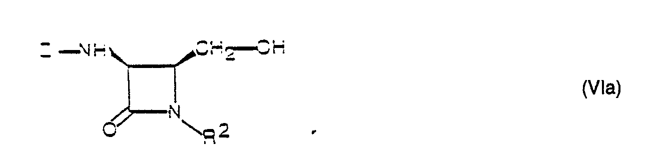 wherein Z and R² have the significance given in claim 1, or of corresponding optical antipodes thereof, characterized by reducing a compound obtained in accordance with claim 21.

## Claims (Claims for the following Contracting State(s) : AT)

. 1. A process for the manufacture of optically uniform β-lactams of the general formula 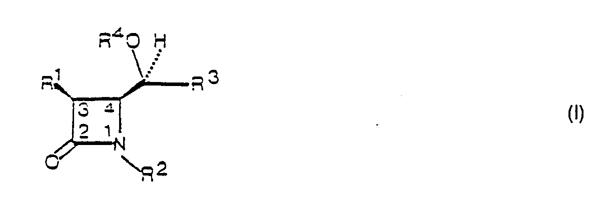 wherein R¹ signifies amino, azido, phthalimido or the group ROCO-CH=C(CH₃)-NH- or Z-NH-, R signifies lower alkyl, Z signifies lower alkoxycarbonyl optionally substituted by chlorine, benzyloxycarbonyl or benzhydryl, R² signifies hydrogen or the group Z-, Z signifies 2,4- or 3,4-di (lower alkoxy) benzyl, 2,4- or 3,4-di(lower alkoxy)phenyl, di[4-(lower alkoxy)phenyl]methyl or 4-(lower alkoxy)phenyl, R³ signifies lower alkyl, phenyl-lower alkyl, lower alkoxy-lower alkyl, especially lower alkoxymethyl, and R⁴ signifies lower alkyl or phenyl-lower alkyl or R³ and R⁴ together with the centre of chirality signify a 5- or 6-membered O-heterocycle which optionally contains a further oxygen atom not directly linked with the centre of chirality and which can be substituted, if desired, by lower alkyl, lower alkoxy, oxo or spirocyclo-lower alkyl, whereby adjacent lower alkoxy groups can together form a ring, such as e.g. in the case of isopropylidenedioxy, whereby R' signifies a group of the formula Z-NH- when R² signifies hydrogen, and the residues denoted by lower have up to 7 carbon atoms, and of coirresponding optical antipodes thereof, characterized by reacting a reactive derivative of a carboxylic acid of the general formula 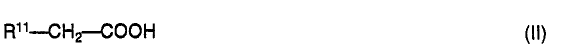 wherein R¹¹ signifies azido, phthalimido or the group ROCO―CH=C(CH₃)―NH― and R signifies lower alkyl, in the presence of a base with an optically uniform compound of the general formula 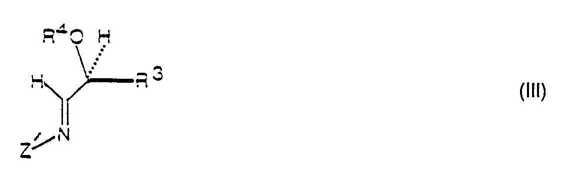 wherein R³, R⁴ and Z' have the above significance, or the optical antipode thereof, if desired converting the group R" in a thus-obtained compound of the general formula 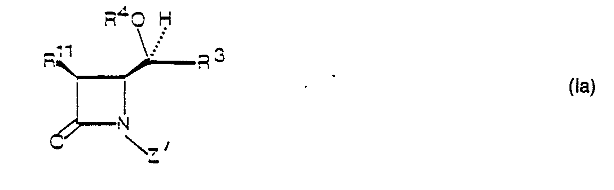 wherein R", R³, R⁴ and Z' have the above significance, or the optical antipode thereof into the amino group, if desired reacting a thus-obtained compound of the general formula 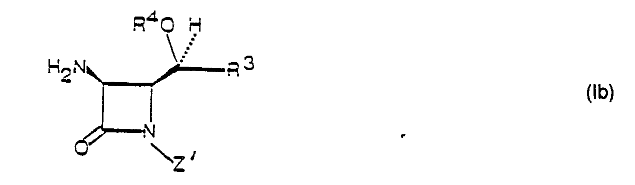 wherein R³, R⁴ and Z' have the above significance, or the optical antipode thereof with an agent yielding the group Z and, if desired, cleaving off the group denoted by Z' in a thus-obtained compound of the general formula 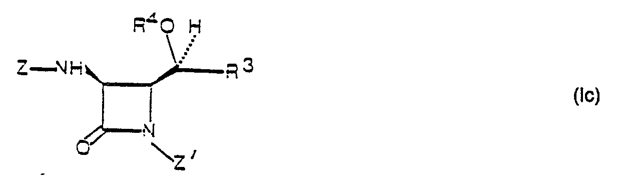 wherein R³, R⁴, Z and Z' have the above significance, or the optical antipode thereof.

2. A process according to claim 1, characterized in that the corresponding carboxylic acid halide, especially the chloride, is used as the reactive derivative of the carboxylic acid of formula 11.

3. A process according to claim 1 or 2, characterized in that there is used a starting compound of formula III or the optical antipode thereof in which R³ and R⁴ together with the centre of chirality signify a group of the formula

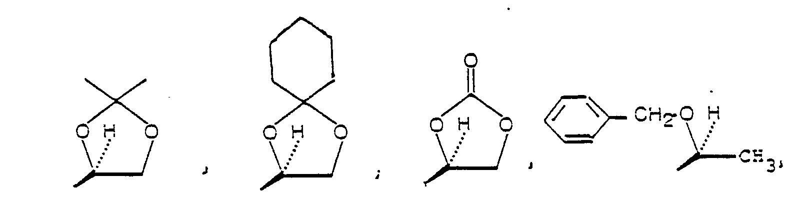

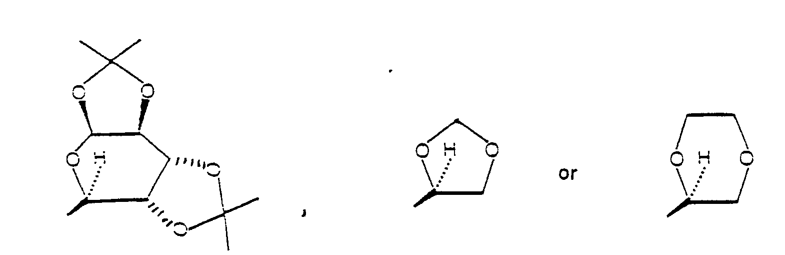

4. A process according to any one of claims 1-3, characterized in that N-[(2R,3R)-1-(2,4-dimethoxybenzyl)-4-[(1,4-dioxaspiro [4.5]dec-2-yl]-2-oxo-3-azetidinyl]-phthalimide is manufactured.

5. A process according to any one of claims 1-3, characterized in that N-[(3S,4S)-cis-1-(3,4-dimethoxybenzyl)-4-[(R)-2,2-dimethyl-1,3-dioxolan-4-yl]-2-oxo-3-azetidinyl]phthalimide is manufactured.

6. A process according to any one of claims 1-3, characterized in that N-[(3S,4S)-4-[(S)-1-(benzyloxy)-ethyl]-1-(2,4-dimethoxybenzyl)-2-oxo-3-azetidinyl]-phthalimide is manufactured.

7. A process according to claim 1, characterized in that R³ and R" together with the centre of chiralitv signify the group

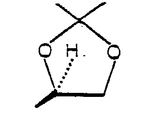

8. A process according to claim 7, characterized in that R¹ signifies phthalimido, amino or benzyloxycarbonylamino and R² signifies 2,4-dimethoxybenzyl.

9. A process according to claim 7 or 8, characterized in that N-[(3S,4S)-cis-1-(2,4-dimethoxybenzyl)-4-[(R)-2,2-dimethyl-1,3-dioxolan-4-yl]-2-oxo-3-azetidinyl]-phthalimide, (3S,4S)-cis-3-amino-1-(2,4-dimethoxybenzyl)-4-[(R)-2,2-dimethyl-1,3-dioxolan-4-yl]-2-azetidinone, benzyl (3S,4S)-cis-1-(2,4-dimethoxybenzyl)-4-[(R)-2,2-dimethyl-1,3-dioxolan-4-yl]-2-oxo-3-azetidinecarbamate and/or benzyl (3S,4S)-cis-4-[(R)-2,2-dimethyl-1,3-dioxolan-4-yl]-2-oxo-3-azetidinecarbamate is manufactured.

10. A process for the manufacture of optically uniform β-lactams of the general formula 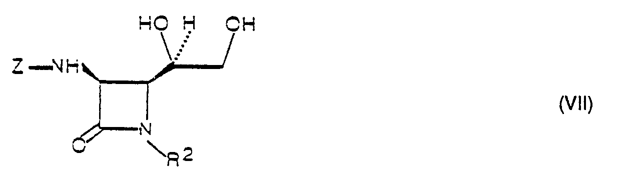 wherein Z and R² have the significance given in claim 1, and of corresponding optical antipodes thereof, characterized by cleaving off the residue R⁵ in a compound obtained in accordance with claim 1 of the general formula 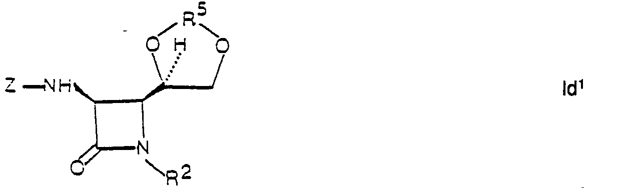 wherein R⁵ signifies methylene, ethylene, oxomethylene, cyclohexylidene or isopropylidene and Z and R² have the above significance, or the optical antipode thereof.

11. A process according to claim 10, characterized in that the compound of formula Id¹ or the optical antipode thereof is treated with a mild acidic agent, e. g. with a sulphonated ion exchanger, pyridinium p-toluenesulphonate or p-toluenesulphonic acid.

12. A process in accordance with claim 10 or 11, characterized in that R⁵ signifies isopropylidene.

13. A process in accordance with any one of claims 10 to 12, characterized in that Z signifies benzyloxycarbonyl and R² signifies hydrogen or 2,4-dimethoxybenzyl.

14. A process in accordance with any one of claims 10 to 13, characterized in that benzyl (3S,4S)-cis-4-[(R)-1,2-dihydroxyethylJ-1-(2,4-dimethoxybenzyl)-2-oxo-3-azetidinecarbamate is manufactured.

15. A process for the manufacture of optically uniform β-lactams of the general formula 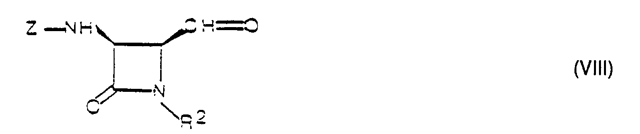 wherein Z and R² have the significance given in claim 1, or of corresponding optical antipodes thereof, characterized by cleaving the diol grouping in a compound obtained in accordance with claim 10.

16. A process according to claim 15, characterized in that the cleavage is carried out with the aid of sodium metaperiodate.

17. A process according to claim 15 or 16, characterized in that benzyl (3S,4S)-cis-1-(2,4-dimethoxybenzyl)-4-formyl-2-oxo-3-azetidinecarbamate is manufactured.

18. A process according to claim 15 or 16, characterized in that benzyl (3S,4S)-cis-4-formyl-2-oxo-3- azetidinecarbamate is manufactured.

19. A process for the manufacture of optically uniform β-lactams of the general formula 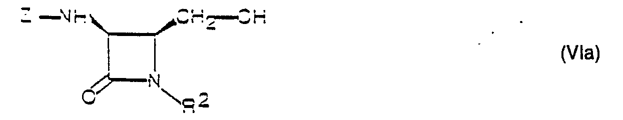 wherein Z and R² have the significance given in claim 1, or of corresponding optical antipodes thereof, characterized by reducing a compound obtained in accordance with claim 15.

20. A process according to claim 19, characterized in that the reduction is carried out with sodium borohydride in a lower alkanol.

21. A process according to claim 19 or 20, characterized in that benzyl (3S,4S)-cis-1-(2,4-dimethoxybenzyl)-4-hydroxymethyl-2-oxo-3-azetidinecarbamate is manufactured.

## Revendications (Revendications pour l'(les) Etat(s) contractant(s) suivant(s) : BE, CH, DE, FR, GB, IT, LI, LU, NL, SE)

1. β-Lactames, a l'etat d'isomeres optiques purs, de formule générale 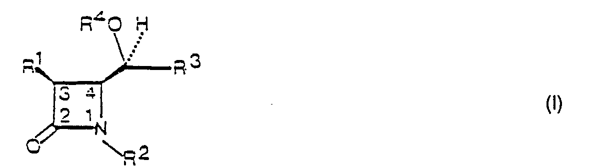 dans laquelle R¹ représente un groupe amino, azido, phtalimido ou un groupe ROCO―CH=C(CH₃)―NH― ou Z―NH―, R représente un groupe alkyle inférieur, Z représente un groupe alcoxycarbonyle inférieur, éventuellement substitué par le chlore, benzyloxycarbonyle ou benzhydryle, R² représente I'hydrogene ou le groupe Z'―, Z' représente un groupe 2,4- ou 3,4-di-(alcoxy inférieur)-benzyle, 2,4- ou 3,4-di-(alcoxy inférieur)phényle, di-[4-(alcoxy inférieur)-phényl]-méthyle ou 4-(alcoxy inférieur)-phényle, R³ représente un groupe alkyle inférieur, -phényl-alkyle inférieur, (alcoxy inférieur- alkyle inférieur, en particulier (alcoxy inférieur)-méthyle et R⁴ représente un groupe alkyle inférieur ou phényl-alkyle inférieur ou bien R³ et R⁴ forment ensemble et avec le centre de chiralité un heterocycle oxygéné a 5 ou 6 chainons contenant éventuellement un autre atome d'oxygene non relié directement avec le centre de chiralité, qui peut le cas échéant être substitué par des groupes alkyle inférieurs, alcoxy inférieurs, oxo ou spirocyclo-alkyle inférieurs, des groupes alcoxy inférieurs voisins pouvant former ensemble un cycle, par exemple un cycle isopropylidènedioxy, étant spécifié que R¹ représente un groupe de formule Z―NH― lorsque R² représente l'hydrogène, et que les groupes qualifies d'inferieurs contiennent jusqu'à 7 atomes de carbone, et les antipodes optiques correspondants.

2. Composes selon la revendication 1, dans lesquels R³ et R⁴ forment ensemble et avec le centre de chiralité un groupe de formule

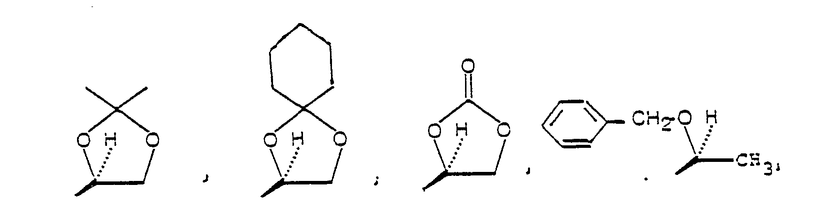

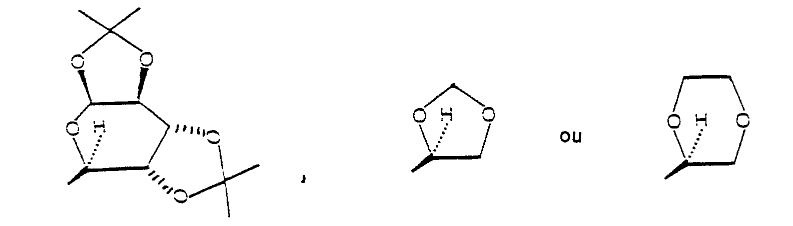

3. Le N-[(2R,3R)-1-(2,4-dimethoxybenzyl)-4-[(S)-1,4-dioxaspiro[4.5]deca-2-yl]-2-oxo-3-azetidinyl]-phtalimide.

4. Le N-[(3S,4S)-cis-1-(3,4-dimethoxybenzyl)-4-[(R)-2,2-dimethyl-1,3-dioxolanne-4-yl]-2-oxo-3-azetidi- nyl]-phtalimide.

5. Le N-[(3S,4S)-4-[(S)-1-(benzyloxy)-ethyl]-1-(2,4-dimethoxybenzyl)-2-oxo-3-azetidinyl]-phtalimide.

6. Composes selon la revendication 1, dans lesquels R³ et R⁴ forment ensemble et avec le centre de chiralité le groupe 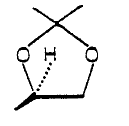 et les antipodes optiques correspondants.

7.Composés selon la revendication 6, dans lesquels R¹ représente un groupe phtalimido, amino ou benzyloxycarbonylamino et R² représente l'hydrogène ou un groupe 2,4-diméthoxybenzyle.

8. Le N-[(3S,4S)-cis-1-(2,4-diméthoxybenzyl)-4-[(R)-2,2-diméthyl-1,3-dioxolanne-4-yl]-2-oxo-3-azétidi- nyl]-phtalimide.

9. La (3S,4S)-cis-3-amino-1-(2,4-diméthoxybenzyl)-4-[(R)-2,2-diméthyl-1,3-dioxolanne-4-yl]-2-azétidi- none.

10. Le (3S,4S)-cis-1-(2,4-diméthoxybenzyl)-4-[(R)-2,2-diméthyl-1,3-dioxolanne-4-yl]-2-oxo-3-azétidi- ne-carbamate de benzyle.

11. Le (3S,4S)-cis-4-[(R)-2,2-diméthyl-1,3-dioxolanne-4-yl]-2-oxo-3-azétidine-carbamate de benzyle.

12. β-Lactames, a l'état d'isomeres optiques purs, de formule générale 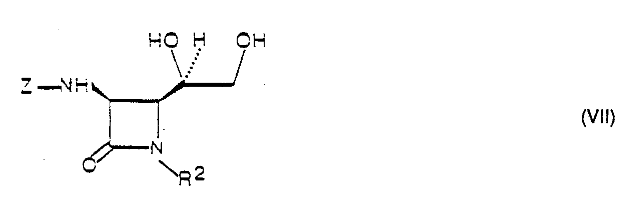 dans laquelle Z et R² ont les significations indiquées dans la revendication 1, et les antipodes optiques correspondants.

13. Composes selon la revendication 12, dans lesquels Z représente un groupe benzyloxycarbonyle et R² I'hydrogene ou un groupe 2,4-dimethoxybenzyle.

14. Le (3S,4S)-cis-4-[(R)-1,2-dihydroxyéthyl]-1-(2,4-diméthoxybenzyl)-2-oxo-3-azétidine-carbamate de benzyle.

15. β-Lactames, a l'état d'isomères optiques purs, de formule générale 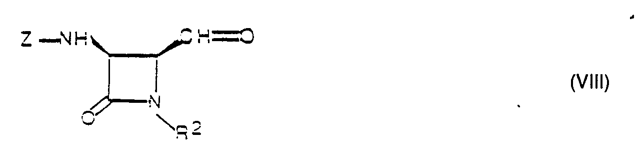 dans laquelle Z et R² ont les significations indiquées dans la revendication 1, et les antipodes optiques correspondants.

16. Composes selon la revendication 15, dans lesquels Z représente un groupe benzyloxycarbonyle et R² I'hydrogene ou un groupe 2,4-diméthoxybenzyle.

17. Le (3S,4S)-cis-1-(2,4-diméthoxybenzyl)-4-formyl-2-oxo-3-azétidine-carbamate de benzyle.

18. Le (3S,4S)-cis-4-formyl-2-oxo-3-azétidine-carbamate de benzyle.

19. Procédé de preparation des composes selon l'une des revendications 1 à 11, caractérisé en ce que I'on fait réagir un derive réactif d'un acide carboxylique de formule générale 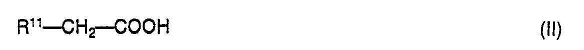 dans laquelle R¹¹ représente un groupe azido, phtalimido ou le groupe ROCO―CH=C(CH₃)―NH― et R représente un groupe alkyle inférieur, en presence d'une base, avec un compose a l'état d'isomère optique pur, de formule générale 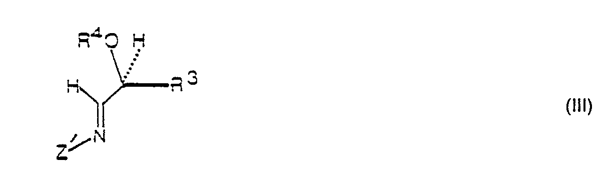 dans laquelle R³, R⁴ et Z' ont les significations indiquées dans la revendication 1, ou I'antipode optique de ce compose, et si on le desire, dans un compose ainsi obtenu, répondant à la formule générale 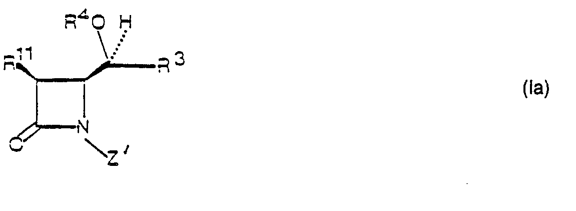 dans laquelle R¹¹, R³, R⁴ et Z' ont les significations indiquées ci-dessus, ou dans I'antipode optique de ce compose, on convertit le groupe R¹¹ en le groupe amino, et si on le desire, on fait réagir un compose ainsi obtenu, répondant a la formule générale 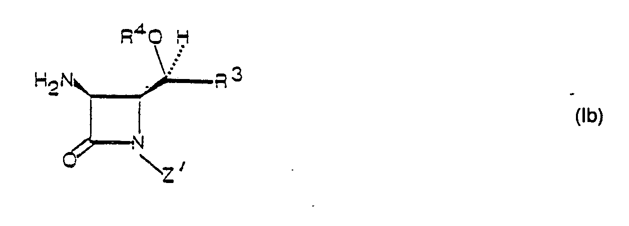 dans laquelle R³, R⁴ et Z' ont les significations indiquées ci-dessus, ou I'antipode optique de ce compose, avec un agent apportant le groupe Z, et si on le desire, dans un compose ainsi obtenu, répondant à la formule générale 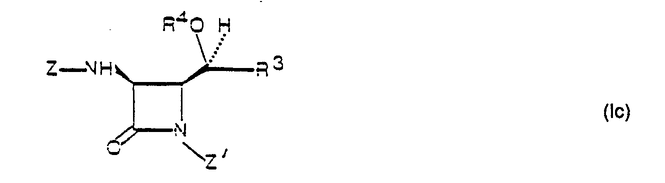 dans laquelle R³, R⁴ et Z' ont les significations indiquées ci-dessus et Z a les significations indiquées dans la revendication 1, ou dans I'antipode optique de ce compose, on élimine le groupe représenté par Z'.

20. Procédé de preparation de β-lactames, a l'état d'isomeres optiques purs, de formule générale 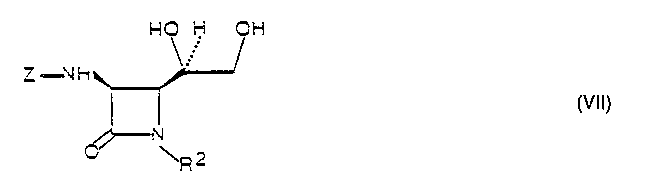 dans laquelle Z et R² ont les significations indiquées dans la revendication 1, et des antipodes optiques correspondants de ces composes, caractérisé en ce que, dans un compose obtenu selon la revendication 19 et répondant a la formule générale 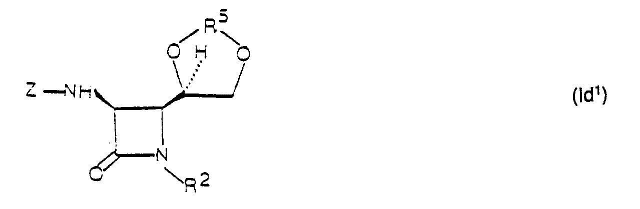 dans laquelle R⁵ représente un groupe methylene, ethylene, oxométhylène, cyclohexylidene ou isopropylidene, et Z et R² ont les significations indiquées ci-dessus, ou dans I'antipode optique de ce compose, on élimine le reste R⁵.

21. Procédé de preparation de β-lactames, a l'état d'isomeres optiques purs, de formule generate 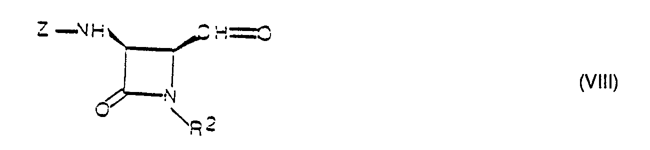 dans laquelle Z et R² ont les significations indiquées dans la revendication 1, et des antipodes optiques correspondants de ces composes, caractérisé en ce que, dans un compose obtenu selon la revendication 20, on élimine le groupement diol.

22. Procédé de preparation de β-lactames, a l'état d'isomères optiques purs, de formule générale 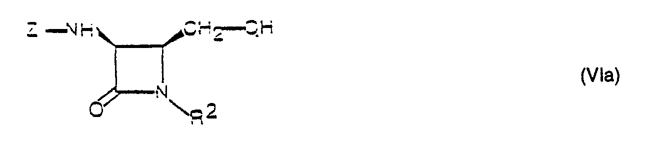 dans laquelle Z et R² ont les significations indiquées dans la revendication 1, et des antipodes optiques correspondants de ces composes, caractérisé en ce que I'on réduit un compose obtenu selon la revendication 21.

## Revendications (Revendications pour l'(les) Etat(s) contractant(s) suivant(s) : AT)

1. Procédé de préparation de β-lactames, à l'état d'isomères optiques purs, de formule générale 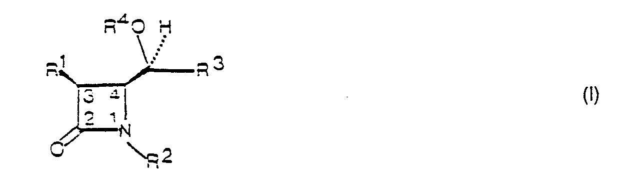 dans laquelle R¹ représente un groupe amino, azido, phtalimido ou le groupe ROCO-CH=C(CH₃)-NH-ou Z-NH-, R représente un groupe alkyle inférieur, Z représente un groupe alcoxycarbonyle inférieur éventuellement substitué par le chlore, benzyloxycarbonyle ou benzhydryle, R² représente l'hydrogène ou le groupe Z'-, Z' représente un groupe 2,4- ou 3,4-di-(alcoxy inférieur)-benzyle, 2,4- ou 3,4-di-(alcoxy inférieur)-phényle, di-[4-(alcoxy inférieur)-phényl]-méthyle ou 4-(alcoxy inférieur)-phényle, R³ représente un groupe alkyle inférieur, phényl-alkyle inférieur, (alcoxy inférieur)-alkyle inférieur, en particulier (alcoxy inférieur)-méthyle, et R⁴ représente un groupe alkyle inférieur ou phényl-alkyle inférieur ou bien R³ et R⁴ forment ensemble et avec le centre de chiralité un hétérocycle oxygéné à 5 ou 6 chaînons, contenant le cas échéant un autre atome d'oxygène non relié directement au centre de chiralité, et qui peut être substitué éventuellement par des groupes alkyle inférieurs, alcoxy inférieurs, oxo ou spirocyclo-alkyle inférieurs, des groupes alcoxy inférieurs voisins pouvant former ensemble un cycle, par exemple un cycle isopropylidène-dioxy, étant spécifié que R¹ représente un groupe de formule Z-NH- lorsque R² représente l'hydrogène, et que les groupes qualifiés d'inférieurs contiennent jusqu'à 7 atomes de carbone, et des antipodes optiques correspondants de ces composés, caractérisé en ce que l'on fait réagir un dérivé réactif d'un acide carboxylique de formule générale 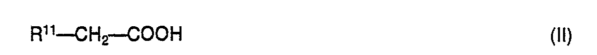 dans laquelle R¹¹ représente un groupe azido, phtalimido ou le groupe ROCO―CH=C(CH₃)―NH― et R un groupe alkyle inférieur, en présence d'une base, avec un composé, à l'état d'isomère optique pur, de formule générale 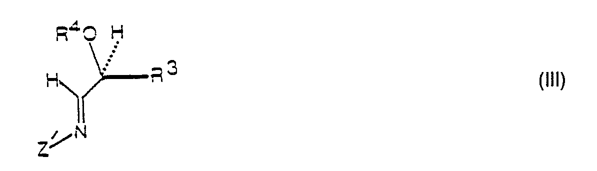 dans laquelle R³, R⁴ et Z' ont-les significations indiquées ci-dessus, ou avec l'antipode optique de ce composé, et si on le désire, dans un composé ainsi obtenu, répondant à la formule générale 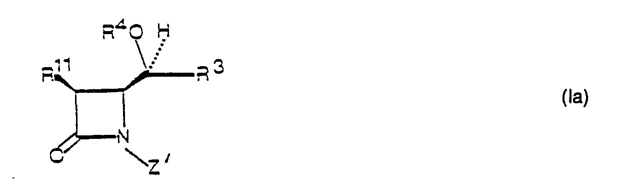 dans laquelle R", R³, R⁴ et Z' ont les significations indiquées ci-dessus, ou dans son antipode optique, on convertit le groupe R¹¹ en le groupe amino, et si on le désire, on fait réagir un composé ainsi obtenu, répondant à la formule générale 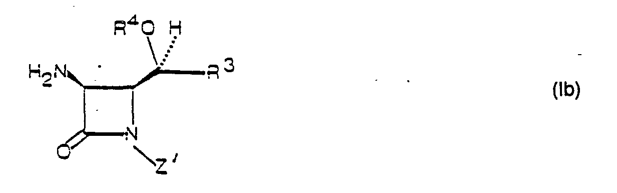 dans laquelle R³, R⁴ et Z' ont les significations indiquées ci-dessus, ou son antipode optique, avec un agent apportant le groupe Z, et si on le désire, dans un composé ainsi obtenu, répondant à la formule générale 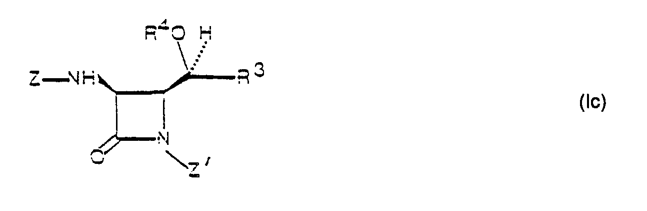 dans laquelle R³, R⁴, Z et Z' ont les significations indiquées ci-dessus, ou dans son antipode optique, on élimine le groupe représenté par Z'.

2. Procédé selon la revendication 1, caractérisé en ce que l'on utilise en tant que dérivé réactif de l'acide carboxylique de formule Il un halogénure de l'acide carboxylique correspondant, en particulier le chlorure.

3. Procédé selon la revendication 1 ou 2, caractérisé en ce que l'on utilise un composé de départ de formule III ou son antipode optique, dans lesquels R³ et R⁴ forment ensemble et avec le centre de chiralité, un groupe de formule

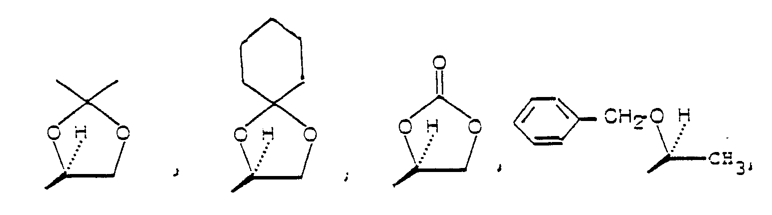

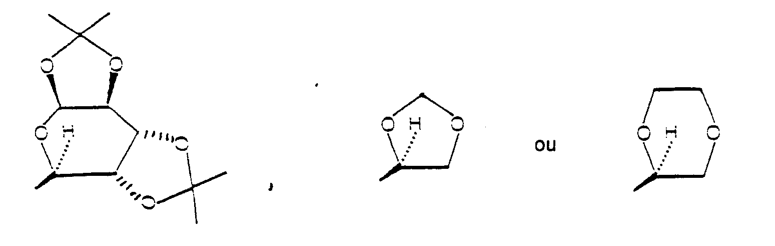

4. Procédé selon l'une des revendications 1 à 3, caractérisé en ce que l'on prépare le N-[(2R,3R)-1-(2,4-diméthoxybenzyl)-4-[(S)-1,4-dioxaspiro[4.5]déca-2-yl]-2-oxo-3-azétidinyl]-phtalimide.

5. Procédé selon l'une des revendications 1 à 3, caractérisé en ce que l'on prépare le N-[(3S,4S)-cis-1-(3,4-diméthoxybenzyl)-4-[(R)-2,2-diméthyl-1,3-dioxolanne-4-yl]-2-oxo-3-azétidinyl]-phtalimide.

6. Procédé selon l'une des revendications 1 à 3, caractérisé en ce que l'on prépare le N-[(3S,4S)-4-[(S)-1-(benzyloxy)-éthyl]-1-(2,4-diméthoxybenzyl)-2-oxo-3-azétidinyl]-phtalimide.

7. Procédé selon la revendication 1, caractérisé en ce que R³ et R⁴ forment ensemble et avec le centre de chiralité le groupe

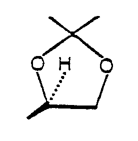

8. Procédé selon la revendication 7, caractérisé en ce que R¹ représente un groupe phtalimido, amino ou benzyloxycarbonylamino et R² un groupe 2,4-diméthoxybenzyle.

9. Procédé selon la revendication 7 ou 8, caractérisé en ce que l'on prépare le N-[(3S,4S)-cis-1-(2,4-diméthoxybenzyl)-4-[(R)-2,2-diméthyl-1,3-dioxolanne-4-yl]-2-oxo-3-azétidinyl]-phtailmide, la (3S,4S)-cis-3-amino-1-(2,4-diméthoxybenzyl)-4-[(R)-2,2-diméthyl-1,3-dioxolanne-4-yl]-2-azétidinone, le (3S,4S)-cis-1-(2,4-diméthoxybenzyl)-4-[(R)-2,2-diméthyl-1,3-dioxolanne-4-yl]-2-oxo-3-azétidine-carbamate de benzyle et/ou le (3S,4S)-cis-4-((R)-2,2-diméthyl-1,3-dioxolanne-4-yl]-2-oxo-3-azétidine-carbamate de benzyle.

10. Procédé de préparation de ß-lactames, à l'état d'isomères optiques purs, de formule générale 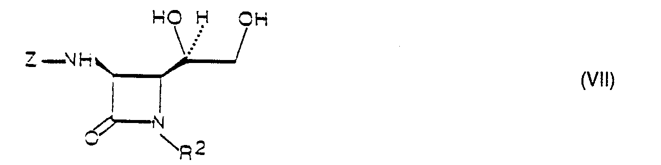 dans laquelle Z et R² ont les significations indiquées dans la revendication 1, et des antipodes optiques correspondants de ces composés, caractérisé en ce que, dans un composé obtenu selon la revendication 1, répondant à la formule générale 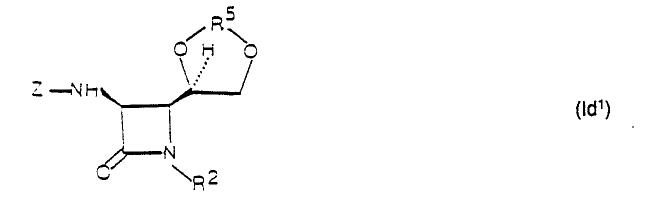 dans laquelle R⁵ représente un groupe méthylène, éthylène, oxométhylène, cyclohexylidène ou isopropylidène et Z et R² ont les significations indiquées ci-dessus, ou dans son antipode optique, on élimine le reste R⁵.

11. Procédé selon la revendication 10, caractérisé en ce que l'on traite le composé obtenu répondant à la formule Id' ou son antipode optique par un agent à acidité modérée, par exemple un échangeur d'ions sulfoné, du p-toluène-suifonate de pyridinium ou de l'acide p-toluène-sulfonique.

12. Procédé selon la revendication 10 ou 11, caractérisé en ce que R⁵ représente un groupe isopropylidène.

13. Procédé selon l'une des revendications 10 à 12, caractérisé en ce que Z représente un groupe benzyloxycarbonyle et R² l'hydrogène ou un groupe 2,4-diméthoxybenzyle.

14. Procédé selon l'une des revendications 10 à 13, caractérisé en ce que l'on prépare le (3S, 4S)-cis-4-[(R)-1,2-dihydroxyéthyl]-1-(2,4-diméthoxybenzyl)-2-oxo-3-azétidine-carbamate de benzyle.

15. Procédé de préparation de β-lactames, à l'état d'isomères optiques purs de formule générale 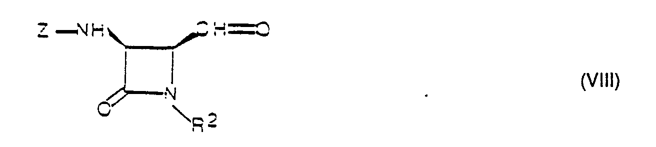 dans laquelle Z et R² ont les significations indiquées dans la revendication 1, et des antipodes optiques correspondants de ces composés, caractérisé en ce que, dans un composé obtenu selon la revendication 10, on élimine le groupement diol.

16. Procédé selon la revendication 15, caractérisé en ce que l'élimination est réalisée à l'aide du métaperiodate de sodium.

17. Procédé selon la revendication 15 ou 16, caractérisé en ce que l'on prépare le (3S,4S)-cis-1-(2,4-diméthoxybenzyl)-4-formyl-2-oxo-3-azétidine-carbamate de benzyle.

18. Procédé selon la revendication 15 ou 16, caractérisé en ce que l'on prépare le (3S,4S)-cis-4- formyl-2-oxo-3-azétidine-carbamate de benzyle.

19.Procédé de préparation de β-lactames, à l'état d'isomères optiques purs de formule générale 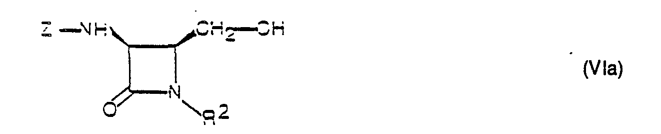 dans laquelle Z et R² ont les significations indiquées dans la revendication 1, et des antipodes optiques correspondants de ces composés, caractérisé en ce que l'on réduit un composé obtenu selon la revendication 15.

20. Procédé selon la revendication 19, caractérisé en ce que la réduction est effectuée à l'aide du borohydrure de sodium dans un alcanol inférieur.

21. Procédé selon la revendication 19 ou 20, caractérisé en ce que l'on prépare le (3S,4S)-cis-1-(2,4-diméthoxybenzyl)-4-hydroxyméthyl-2-oxo-3-azétidinecarbamate de benzyle.
